Europäisches Patentamt

European Patent Office

Office européen des brevets

(11) Publication number: **0 183 350 B1**

## EUROPEAN PATENT SPECIFICATION

(45) Date of publication of patent specification: **15.01.92**  (51) Int. Cl.⁵: **C12N 15/27**, C12P 21/02, C12N 15/81

(21) Application number: **85306827.8**

(22) Date of filing: **25.09.85**

(54) **DNA Encoding human colony stimulating factor, peptide encoded thereby,vectors and transformed hosts containing such DNA, and the production of all thereof.**

(30) Priority: **29.10.84 US 666041**
**02.07.85 US 750401**

(43) Date of publication of application:
**04.06.86 Bulletin 86/23**

(45) Publication of the grant of the patent:
**15.01.92 Bulletin 92/03**

(84) Designated Contracting States:
**AT BE CH DE FR GB IT LI LU NL SE**

(56) References cited:
**EP-A- 0 116 201**
**WO-A-86/00639**
**WO-A-86/03225**
**US-A- 4 438 032**

**NATURE, vol. 298, 1st July 1982, pages 75-77, Macmillan Journals Ltd; A.J. LUSIS et al.: "Translation of mRNA for human granulocyte-macrophage colony stimulating factor"**

(73) Proprietor: **IMMUNEX CORPORATION**
**Immunex Building 51 University Street**
**Seattle, WA 98101(US)**

(72) Inventor: **Anderson, Dirk M**
**1757 N.W. 58th Street**
**Seattle Washington 98107(US)**
Inventor: **Cantrell,Michael A**
**1056 N.E. 88th Street Seattle**
**Washington 98105(US)**
Inventor: **Cerretti, Douglas P**
**2415 W. Bertona**
**Seattle Washington 98199(US)**
Inventor: **Cosman, David J**
**310-12th Avenue East**
**Seattle Washington 98102(US)**
Inventor: **Larsen, Alf D**
**320 Summit Avenue East, No. 15**
**Seattle Washington 98102(US)**
Inventor: **Price, Virginia L**
**2617 Boyer Avenue East**
**Seattle Washington 98102(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**PROCEEDINGS OF THE NATIONAL ACADEMY OF SCIENCES OF THE USA, vol. 82, no. 18, September 1985, pages 6250-6254, Washington, US; M.A. CANTRELL et al.: "Cloning, sequence, and expression of a human granulocyte/macrophage colony-stimulating factor"**

(74) Representative: **Sheard, Andrew Gregory et al Kilburn & Strode 30, John Street London WC1N 2DD(GB)**

## Description

The present invention relates to a colony stimulating factor (hereinafter "CSF") and, more particularly, to the cloning of the gene for the human granulocyte-macrophage colony stimulating factor (hereinafter "GM-CSF") by use of a nucleotide probe derived from a murine GM-CSF complementary deoxyribonucleic acid ("cDNA") clone to screen a cDNA library synthesized from messenger ribonucleic acid ("mRNA") containing human GM-CSF mRNA, and to the characterization of the GM-CSF gene.

CSF refers to a family of lymphokines which induce progenitor cells found in the bone marrow to differentiate into specific types of mature blood cells. The particular type of mature blood cell that results from a progenitor cell depends upon the type of CSF present. For instance, erythropoietin is believed to cause progenitor cells to mature into erythrocytes while thrombopoietin is thought to drive progenitor cells along the thrombocytic pathway. Similarly, granulocyte-macrophage colony formation is dependent on the presence of GM-CSF. The present invention concerns the cloning of human GM-CSF.

CSF, including human GM-CSF, is produced only in minute quantities in vivo. CSF-like factors have been extracted from body organs, Sheridan and Stanley, 78 J. Cell Physiol. 451-459 (1971), and have been detected in serum and urine, Robinson et al., 69 J. Cell. Physiol. 83-92 (1967); Stanley et al., 79 J. Lab. Clin. Med. 657-668 (1972). Researchers have reported isolating low titer CSF-like factor from human peripheral blood cells which appear to be macrophages or monocytes, Moore and Williams, 80 J. Cell. Physiol. 195-206 (1972); Golde and Kline, 51 J. Clin. Invest. 2981-2983 (1972); Moore et al., 50 J. Natl. Cancer Inst. 591-601 (1973).

Although the factors identified by the above researchers have been reported to be CSF, heretofore sufficient quantities of homogeneous human CSF, including GM-CSF, have not been available to thoroughly investigate its biochemistry and biology. The availability of adequate quantities of homogeneous human GM-CSF would be valuable in investigations and possible treatment of proliferative blood disorders, such as certain leukemias and anemias. Also, human GM-CSF in greater purity and larger quantities than heretofore available, could prove useful in achieving successful bone marrow transplantation following cancer chemotherapy.

One potential method of providing relatively large quantities of homogeneous human GM-CSF is through recombinant DNA techniques. Recombinant DNA techniques have been developed for economically producing a desired protein once the gene coding for the protein has been isolated and identified. A discussion of such recombinant DNA techniques for protein production is set forth in the editorial and supporting papers in Vol. 196 of Science (April 1977). However, to take advantage of the recombinant DNA techniques discussed in this reference, the gene coding for human GM-CSF must first be isolated.

In accordance with the present invention, the gene coding for human GM-CSF is isolated from a cDNA library with a nick-translated cDNA probe. The probe is isolated from a murine GM-CSF cDNA library by use of a synthetic oligonucleotide probe corresponding to a portion of the nucleotide sequence of murine GM-CSF. Total human RNA is extracted from cell lines or other sources thought to produce relatively high levels of GM-CSF. Polyadenylated mRNA is isolated from the total RNA extract. A cDNA library is constructed by reversed transcription of the polyadenylated mRNA with reverse transcriptase. The DNA is rendered double-stranded with DNA polymerase I and inserted into an appropriate cloning vector. Resultant recombinant cloning vectors are used to transform an appropriate host.

Transformed hosts are identified and grouped into pools. Plasmid DNA prepared from these pools is hybridized with the murine cDNA probe that has been radiolabeled. The pool(s) of clones that give a positive signal to the probe is identified and then the putative pool subdivided and the hybridization screen repeated. A single transformant corresponding to the human GM-CSF gene is eventually identified. Plasmid DNA is prepared from this transformant and characterized by DNA sequencing. In addition, the corresponding amino acid sequence is determined from the nucleotide sequence. The coding region of the human GM-CSF gene is cloned in a yeast host system to express mature GM-CSF. Thereafter biological assays are conducted to confirm that the expressed protein product is GM-CSF.

The details of typical embodiments of the present invention will be described in connection with the accompanying drawings, in which:

FIGURE 1 illustrates the amino acid and nucleotide sequence of the gene coding for murine GM-CSF with the portion of such gene employed as a probe for screening a human cDNA library indicated in solid underline;

FIGURE 2 illustrates the amino acid and nucleotide sequence of the human GM-CSF gene, including the 3' noncoding region;

FIGURE 3 illustrates the pYαfGM-2 expression plasmid with the coding region of the GM-CSF gene inserted therein for use in transforming host cells to express functional human GM-CSF;

FIGURE 4 illustrates Northern blot analysis of GM-CSF mRNA from various cell sources; and,
FIGURE 5 illustrates Southern blot analysis of human genomic DNA.

Sources of Human CSF Producing Cells

Preferably, a cDNA library, from which the gene coding for human GM-CSF will be sought, is constructed from cells previously found to produce relatively high levels of other lymphokines, under the assumption that they might also produce human GM-CSF. These sources may include malignant cell lines, such as a human lymphoma T-cell line. Applicants have prepared cDNA libraries from several human lymphoma T-cell lines, such as HUT-102 and Jurkat. These particular cell lines are available from a wide variety of sources and have been used extensively by researchers. See, for instance, Leonard et al., 80 Proc. Natl. Acad. Sci. U.S.A. 6957 (1983), and Leonard et al., 300 Nature (London) 267 (November 1982).

Activated human peripheral blood mononuclear cells also potentially may be a source of GM-CSF molecules. For use in the present invention, the peripheral blood mononuclear cells can be separated from whole blood by standard techniques, such as by Ficoll-Hypaque centrifugation. Adherent cells are removed by plastic adherence and the remaining leukocytes multiplied by culturing in vitro in a serum containing medium together with a T-cell mitogen.

Applicants have investigated a number of human cell lines, including those noted above, and peripheral blood T-cells as sources for the gene coding for human GM-CSF. As set forth infra, applicants have successfully isolated this gene from cDNA libraries prepared from the HUT-102 cell line and from mitogen activated, nonadherent leukocytes.

Preparation of RNA from Human CSF Producing Cells

Total RNA from human potentially GM-CSF-producing cells is extracted by standard methods, such as disclosed by Chirgwin et al., 18 Biochemistry 5294 (1979), and Maniatis et al., Molecular Cloning, a Laboratory Manual, Cold Spring Harbor Laboratory, Cold Spring Harbor, New York (1982).

As is well known, when extracting RNA from cells, it is important to minimize ribonuclease ("RNase") activity during the initial stages of extraction. One manner in which this is accomplished is to denature the cellular protein, including the RNase, at a rate that exceeds the rate of RNA hydrolysis by RNase. In the procedures of Chirgwin et al., supra, and Maniatis et al., supra at 196, this is carried out by use of guanidinium thiocyanate, together with a reducing agent, such as 2-mercaptoethanol (to break up the protein disulfide bonds). The RNA is isolated from the protein by standard techniques, such as phenol/chloroform extraction, ethanol precipitation or sedimentation through cesium chloride.

Next, polyadenylated mRNA is separated from the extracted protein. Although several techniques have been developed to carry out this separation process, one preferred method is to chromatograph the polyadenylated mRNA on oligo (dT)-cellulose as described by Edmonds et al., 68 Proc. Natl. Acad. Sci. 1336 (1971); Aviv and Leder, 69 Proc. Natl. Acad. Sci. 1408 (1972); and Maniatis et al., supra at 197. The oligo (dT)-cellulose column is prepared with a loading buffer and then the mRNA applied to the column. Thereafter, the column is initially washed with a buffer solution to remove the unpolyadenylated mRNA and then the polyadenylated mRNA is eluted from the column with a buffered, low ionic strength eluent. The integrity of the polyadenylated mRNA is verified by gel electrophoresis.

Preparation of cDNA from mRNA

A library of double-stranded cDNA corresponding to the total mRNA, as prepared above, is constructed by known techniques employing the enzyme reverse transcriptase. One such procedure which may be employed in conjunction with the present invention is detailed by Maniatis et al., supra at 230. Briefly, the polyadenylated mRNA is reverse transcribed by using oligo-dT, that has been hybridized to the polyadenylated tail of the mRNA, as a primer for a first cDNA strand. This results in a "hairpin" loop at the 3' end of the initial cDNA strand that serves as an integral primer for the second DNA strand. Next, the second cDNA strand is synthesized using the enzyme DNA polymerase I and the hairpin loop is cleaved by S1 nuclease to produce double-stranded cDNA molecules. The double-stranded cDNA is fractionated by any convenient means to remove the shorter strands, thereby avoiding the needless cloning of small cDNA fractions.

It is to be understood that in accordance with the present invention, alternative standard procedures may be employed to prepare double-stranded cDNA from mRNA. One such alternative technique is disclosed by Land et al., 9 Nucl. Acids Res. 2251 (1981). In the Land et al. protocol, the hairpin loop is not used as a primer for the second cDNA strand. Rather, the 3' end of the first cDNA strand is tailed with dCMP residues using terminal deoxynucleotidyl transferase ("TdT"). This produces a 3' tail of poly-C residues. Then the synthesis of the second strand is primed by oligo-dG hybridized to the 3' tail. This technique is said to help avoid losing portions of the 5' tail of the second cDNA strand which might occur if the hairpin is cleaved with S1 nuclease, as in the Maniatis et al. protocol.

Cloning of cDNA

Next, the double-stranded cDNA is inserted within a cloning vector which is used to transform compatible prokaryotic or eukaryotic host cells for replication of the vector. Thereafter, the transformants are identified and plasmid DNA prepared therefrom.

To carry out the present invention, various cloning vectors may be utilized. Although the preference is for a plasmid, the vector may be a bacteriophage or a cosmid. If cloning occurs in mammalian cells, viruses also can be used as vectors.

If a plasmid is employed, it may be obtained from a natural source or artificially synthesized. The particular plasmid chosen should be compatible with the contemplated transformation host, whether a bacteria such as Escherichia coli ("E. coli"), yeast, or other unicellular microorganism. The plasmid should have the proper origin of replication for the particular host cell to be employed. Also, the plasmid should have a phenotypic property that will enable the transformed host cells to be readily identified and separated from cells that do not undergo transformation. Such phenotypic characteristics can include genes providing resistance to growth inhibiting substances, such as an antibiotic. Plasmids are commercially available that encode genes resistant to various antibiotics, including tetracycline, streptomycin, sulfa drugs, penicillin, and ampicillin.

If E. coli is employed as the host cell, many possible cloning plasmids are commercially available which may be used in conjunction with the present invention. A preferred plasmid for performing the present invention is pBR322. This plasmid has been fully sequenced, as set forth in Sutcliffe, 43 Cold Spring Harbor Symp. Quant. Biol. 77 (1979). A significant advantage of this plasmid is that it has 11 known unique restriction sites, including the Pst I site in the ampicillin resistant gene. This feature is particularly useful for cloning by the homopolymer tailing method.

If a bacteriophage is used instead of a plasmid, such phages should have substantially the same characteristics noted above for selection of plasmids. This includes the existence of a phenotypic marker and ligatable termini for attachment of foreign genes.

Preferably, in the present invention, the double-stranded cDNA, having blunt ends, is inserted into a plasmid vector by homopolymeric tailing. As is well known in the art, in this technique, complementary homopolymer tracks are added to the strands of the cDNA and to the plasmid DNA. The vector and double-stranded cDNA are then joined together by hydrogen bonding between complementary homopolymeric tails to form open, circular hybrid molecules capable of transforming host cells, such as E. coli.

In one procedure for homopolymeric tailing, approximately 50 to 150 dA nucleotide residues are added to the 3' ends of linearized plasmid DNA. A similar number of dT nucleotide residues are added to the 3' ends of the double-stranded cDNA and then the cDNA and plasmid joined together.

In an alternative and preferred method, dG tails are added to the 3' ends of the cloning vector that has been cleaved with an appropriate restriction enzyme. For instance, if the pBR322 plasmid is employed, the restriction enzyme Pst I may be used to digest the plasmid at the ampicillin resistant gene. Complementary dC tails are added to the 3' ends of the double-stranded cDNA prior to insertion of the cDNA segment in the plasmid with on appropriate annealing buffer.

It is to be understood that the double-stranded cDNA may be inserted within plasmid cloning vectors by other various standard methods. One such alternative technique involves attaching synthesized nucleotide linkers to the ends of the cDNA strands by using DNA ligase. The linkers are cleaved with a restriction enzyme to generate cohesive termini for insertion within a plasmid cleaved with the same restriction enzyme. Scheller et al., 196 Science 177-180 (1977); Maniatus et al., supra at 219.

The recombinant DNA plasmids, as prepared above, are used to transform host cells. Although the host may be any appropriate prokaryotic or eukaryotic cell, it is preferably a well-defined bacteria, such as E. coli or a yeast strain. Such hosts are readily transformed and capable of rapid growth in culture. Other forms of bacteria, such as salmonella or pneumococcus, may be substituted for E. coli. In place of bacteria, other unicellular microorganisms may be employed, for instance, fungi and algae. Whatever host is chosen, it should not contain a restriction enzyme that would cleave the recombinant plasmid.

If E. coli is employed as a host, preferable strains are MM294 and RR1. Protocols for transformation of the MM294 host by a plasmid vector are well known, as set forth in Maniatis et al., supra at 255; and, Hanahan, 166 J. Mol. Biol. 557 (1983). Protocols for transformation of the RR1 host by a plasmid vector are also well known as set forth in Bolivar et al., 2 Gene 95 (1977) and Peacock et al., 655 Biochem. Biophys. Acta. 243(1981). Other strains of E. coli which also could serve as suitable hosts include DH1 (ATCC No. 33849) and C600. These strains and the MM294 and RR1 strains are widely commercially available.

In transformation protocols, including those disclosed by Maniatis et al., supra, and Hanahan, supra, only a small portion of the host cells are actually transformed, due to limited plasmid uptake by the cells. The cells that have been transformed can be identified by placing the cell culture on agar plates containing suitable growth medium and a phenotypic identifier, such as an antibiotic. Only those cells that have the proper resistance gene (e.g., to the antibiotic) will survive. If the recombinant pBR322 plasmid is used to transform E. coli strain MM294, transformed cells can be identified by using tetracycline as the phenotypic identifier.

Preparation of Radiolabeled cDNA Screening Probe

A radiolabeled DNA fragment composed of several hundred base-pairs ("bp") corresponding to a majority of the nucleotide sequence of the gene coding for the murine GM-CSF species is used as a probe to screen the above-prepared human cDNA library. The probe is isolated from a murine cDNA library with a radiolabeled, synthetic oligonucleotide probe corresponding to a portion of the nucleotide sequence of murine GM-CSF.

To isolate the cDNA probe for use in the screening procedure of the present invention, a murine cDNA library is initially prepared from murine mRNA using the procedures set forth above. The mRNA is extracted from a murine cell line known to produce GM-CSF species. Such cell lines may include various T and macrophage cell lines, such as the T-lymphoma cell line LBRM-33 or clones thereof which are radiation-induced splenic lymphoma cell lines from the B-10.BR mouse. This cell line and clones thereof are available from a wide variety of commercial and private sources and has been extensively used by U.S. and foreign researchers. Total RNA from the murine cells is extracted by standard methods as discussed above, for instance, by the use of guanidinium thiocyanate together with 2-mercaptoethanol. Thereafter, polyadenylated mRNA is separated from the extracted protein by chromatography on oligo (dT)-cellulose.

A library of double-stranded cDNA corresponding to the murine mRNA is constructed, as discussed above, by employing reverse transcriptase to form an initial cDNA strand by using the mRNA as a template. Next, the enzyme DNA polymerase I is used to synthesize the second cDNA strand, employing the first strand as a template. The double-stranded cDNA is inserted within a cloning vector which is used to transform compatible host cells for replication of the vector. Preferably, the vector is composed of a plasmid having a number of unique restriction sites, such as the plasmid pBR322. The cDNA prepared from the mRNA may be inserted within this plasmid by homopolymeric tailing, as described above. The recombinant plasmids are used to transform a compatible host, such as a strain of E. coli. Of course, other appropriate hosts may be employed. The host cells that are transformed by the recombinant plasmid are identified with an appropriate standard phenotypic identifier, such as an antibiotic.

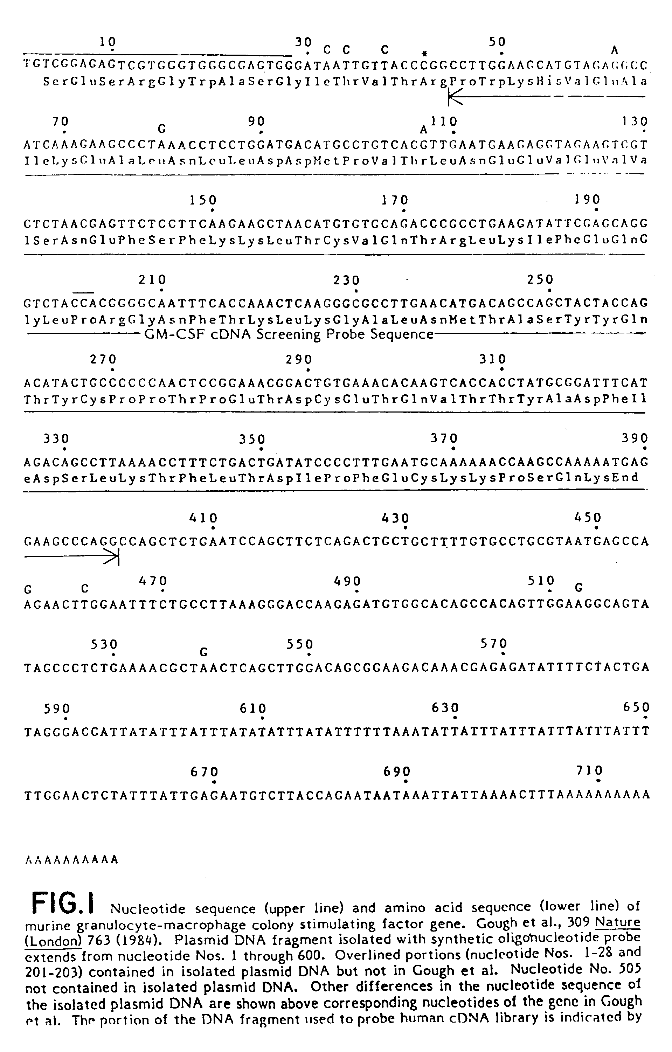

A radiolabeled oligonucleotide is synthesized for use as a probe to screen the murine cDNA library. The probe, derived of a portion of the antisense strand of the gene coding for murine GM-CSF, has the following composition: 5'-TGATGGCCTCTACATGCTTCCAAGGCCGGGTAACAATTAT-3'. This probe complements the 5' terminal portion of the sense strand shown in FIGURE 1, and has the advantage of being short enough to be relatively easily synthesized, while being long enough to contain sufficient information to be useful as a probe for the murine GM-CSF gene. It is to be understood, however, that the composition of the probe may correspond to other portions of the murine GM-CSF gene without departing from the scope or spirit of the present invention.

The synthetic oligonucleotide probe may be readily chemically synthesized by well-known techniques, such as by phosphodiester or triester methods. The details of the triester synthesis technique are set forth, for example, in Sood et al., 4 Nucl. Acid Res. 2557 (1977); and, Hirose et al., 28 Tet. Lett. 2449 (1978). After synthesis, the oligonucleotide probe is labeled with T4 polynucleotide kinase and $^{32}$P-ATP. A standard protocol for the labeling procedure is set forth in Maniatis et al., supra at 122. Advantageously, the oligonucleotide probe can be synthesized with OH 5' termini, thereby avoiding the phosphatase procedure typically required.

The murine cDNA library is screened with the synthetic radiolabeled probe as detailed infra at page 10 and in Examples 3 and 4. Plasmid DNA is then prepared from the particular positive colony identified by the screening procedure.

The murine plasmid DNA is sequenced by the chain-termination method discussed infra at page 11. From the sequencing results, as shown in FIGURE 1, the isolated plasmid DNA was found to include substantially the entire coding region of murine GM-CSF gene, plus additional sequences at the 5' end of the gene and an additional codon, CCA, (nucleotides 201-203), thereby confirming that the murine plasmid DNA isolated from the murine cDNA library codes for murine GM-CSF. Additional differences between the murine GM-CSF gene and the isolated plasmid DNA are shown in FIGURE 1; none of these differences resulted in a change of the encoded amino acid.

Initially the entire length of the isolated murine plasmid DNA was chosen as a probe for screening the human cDNA library prepared above. A relatively large size probe (in the range of 300-500 bp) usually increases the likelihood that cDNA, actually coding for human GM-CSF would be hybridized rather than non-GM-CSF coding cDNA fragments. However, use of this entire murine plasmid DNA insert as a probe was not successful; no hybridization occurred with cDNA fragments of the human library.

Subsequently, the size of the murine probe was reduced and the human cDNA library rescreened. The smaller probe consisted of a fragment extending from nucleotide Nos. 45 to 400 as indicated by the solid underline in FIGURE 1, and thus included only a small portion of the 3' non-coding region and none of the 5' non-coding region of the mouse plasmid DNA fragment. As detailed below, use of this probe was successful in isolating the human GM-CSF gene from a cDNA library. It is to be understood that probes corresponding to other portions of the nucleotide sequence of the murine plasmid DNA fragment may be employed without departing from the spirit or scope of the present invention.

The murine cDNA probe is radiolabeled prior to being used for hybridizing to the human cDNA library pools. Due to the relatively large size of the probe, various labeling techniques may be employed; however, preferably the probe is labeled by "nick translation." In this well-known technique, as discussed by Rigby et al., 113 J. Molec. Bio. 237 (1977), and Maniatis et al., supra at 108, nicks are introduced at widely separated sites in the DNA by very limited treatment with DNase I, thereby exposing a free 3'-OH group at each nick. DNA polymerase I is employed to incorporate appropriate radiolabeled deoxynucleotide triphosphates ($^{32}$P-dNTPs), at the 3'-OH terminus and concurrently remove the nucleotide from the 5' side of the nick causing sequential movement of the nick along the DNA ("nick translation").

Screening of cDNA Library

In the screening procedure of the present invention, the transformants are initially pooled into relatively large groups each composed of approximately 100,000 transformants. The replicated plasmids are extracted from the transformants using any one of several well-known techniques, such as by alkaline lysis. Plasmid DNA is prepared by cleaving the extracted plasmids with Pst I. The resulting DNA segments are fractionated by electrophoresis on agarose gels and then directly analyzed by Southern blotting as described by Southern, 98 J. Mol. Biol. 503 (1975). The DNA fragments that bind to the nitrocellulose filter in the Southern blotting procedure are hybridized with the labeled cDNA probe. The specific DNA fragments that hybridize to the probe are identified by autoradiography.

The putative pool(s) of clones that discloses a strongly hybridizing band during autoradiography is subdivided into groups of approximately 5,000 transformants, and then the above-described hybridizing screen using the labeled murine cDNA probe is repeated. This process of subdividing putative pools of clones and screening transformants is repeated until a desired pool size is obtained. A single transformant that hybridizes to the labeled probe is then identified by the well-known colony hybridizing technique of Grunstein and Hogness, 72 Proc. Natl. Acad. Sci. 3961 (1975). By this procedure, applicants have discovered one such positive colony. Plasmid DNA, designated as pHG23, is prepared from this particular colony.

Characterization of Screened cDNA

The plasmid DNA prepared above is sequenced using standard chain-termination methods. This technique of nucleotide sequencing was originated by Sanger et al., 70 Proc. Natl. Acad. Sci. (USA) 5463 (1977). See U.S. Patent No. 4,322,499. Methods for chain-termination sequence determination are set forth in the Amersham Handbook entitled, M13 Cloning and Sequencing, Blenheim Cresent, London (1983) (hereinafter "Amersham Handbook"); Messing, 2 Recombinant DNA Technical Bulletin, NIH Publication No. 79-99, 2, 43-48 (1979); Norrander et al., 26 Gene 101 (1983); Cerretti et al., 11 Nucl. Acids Res. 2599 (1983); and, Biggin et al., 80 Proc. Natl. Acad. Sci. (USA) 3963 (1983). M13 filamentous phage is employed as a vector to clone the DNA sequence of interest. These phage vectors provide single-stranded DNA templates which are readily sequenced by the chain-termination method, which involves priming a single-stranded template molecule with a short primer strand having a free 3' hydroxyl group and then using DNA polymerase (Klenow fragment) to copy the template strand in a chain extension reaction using all four deoxyribonucleotide triphosphates, i.e., dATP, dCTP, dGTP, and dTTP (collectively referred to as "dNTPs"), with one of the dNTPs being radiolabeled. In the synthesis reaction, a nucleotide specific chain terminator lacking a 3'-hydroxyl terminus, for instance, a 2', 3' dideoxynucleotide triphosphate ("ddNTP"), is used to produce a series of different length chain extensions. The terminator has a normal 5' terminus so that it can be incorporated into a growing DNA chain, but lacks a 3' hydroxyl terminus. Once the terminator has been integrated into a DNA chain, no further deoxynucleotide triphosphates can be added so that growth of the chain stops. Four separate synthesizing reactions are carried out, each having a ddNTP of one of the four nucleotide dNPTs, i.e., dATP, dCPT, dGTP and dTTP. One of the normal dNTPs is radiolabeled so that the synthesized strands, after having been sorted by size on a polyacrylamide gel, can be autoradiographed. The chain extensions from the four reactions are placed side by side in separate gel lanes so that the pattern of the fragments from the autoradiography corresponds to the nucleic acid sequence of the cloned DNA.

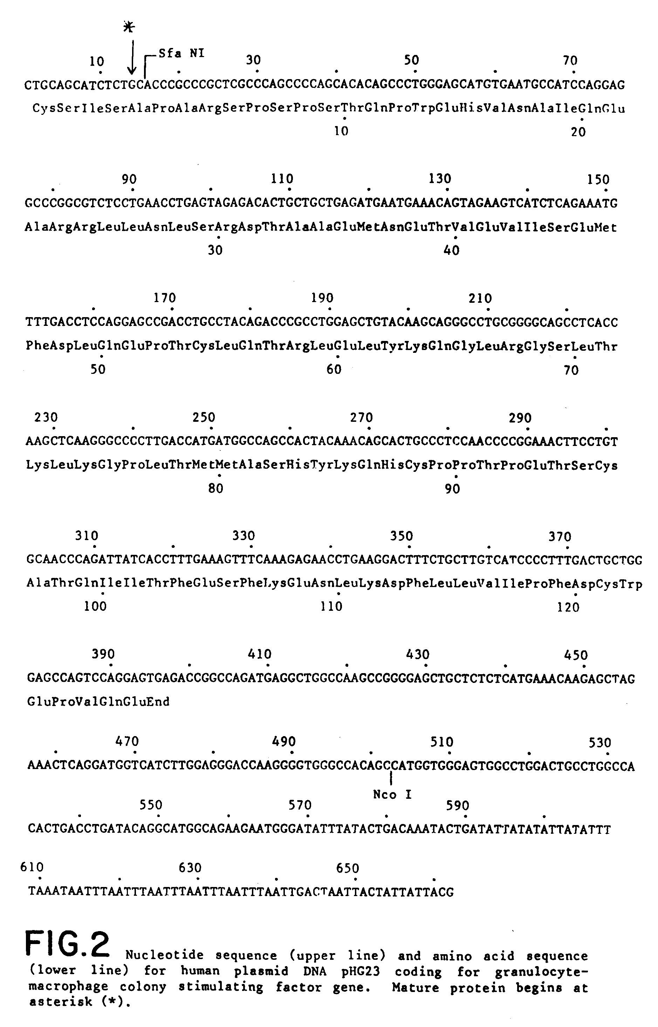

FIGURE 2 illustrates the nucleotide sequence of the human GM-CSF gene contained in the pHG23 plasmid DNA prepared above. The corresponding amino acid composition of the coding region of the gene is also illustrated in FIGURE 2, beginning from the Ala residue, No. 1 (nueleotide No. 14) and extending to the Glu residue, No. 127 (nucleotide No. 394).

In preparation for the sequencing procedures, the plasmid DNA containing the DNA insert is subcloned into M13 phage vectors to form single stranded DNA templates. A universal primer is used to sequence the sense and antisense strands. Rather than relying on the sequencing results obtained from sequencing the entire length of the fragments with a single chain-termination procedure, an additional synthetically produced primer is used to initiate the chain-termination procedure from an intermediate location along the length of the subcloned DNA fragment. The composition of the synthetically produced primer was based on the sequence information obtained using the universal primer. By this process, both strands of the subcloned DNA fragment are sequenced in overlapping fashion, thereby serving to redundantly confirm the sequences.

It is to be understood that rather than employing the chain-termination technique outlined above, other known methods may be utilized to sequence cloned human cDNA inserts without departing from the spirit or scope of the present invention. For instance, the chemical degradation method of Maxam and Gilbert as set forth in 74 Proc. Nat'l Acad. Sci. (USA) 560 (1977) can be used.

Expression of Functional GM-CSF from cDNA Clone

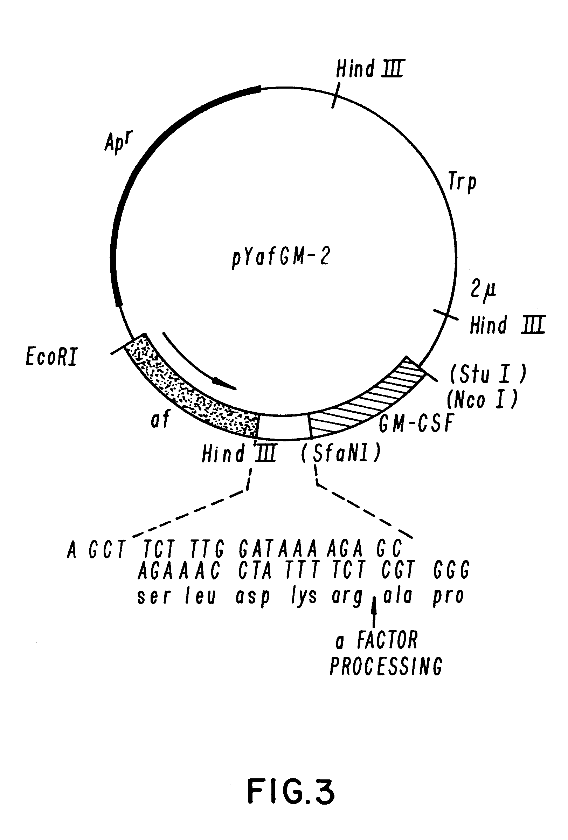

To determine whether the cDNA coding region of the GM-CSF gene as contained in pHG23 would encode functional GM-CSF, the gene is expressed in host cells and then tested for its ability to stimulate the growth of bone marrow colonies in agar. A cDNA fragment of substantially the entire coding region of the GM-CSF gene shown in FIGURE 2, from the Sfa NI to Nco I fragment, is inserted into an expression vector, FIGURE 3, designed to direct synthesis and secretion of the mature form of GM-CSF from yeast host cells. The expression vector, designated as pY α fGM-2, contains sequences derived from plasmid pBR 322 (thick line) containing an origin of replication and the ampicillin resistance gene (Apʳ) The expression vector also includes sequences from yeast (thin line in FIGURE 3), including the tryptophan-1 gene (Trp-1) as a selectable marker and the 2 u yeast origin of replication. The expression vector further includes the yeast pre-pro- α mating factor (" α-factor") as an efficient promoter together with leader sequences to direct the synthesis and secretion of GM-CSF in yeast hosts, followed immediately by the sequence for the coding region of GM-CSF shown in FIGURE 2. The structure of the α -factor gene is discussed in Kurjan and Herskowitz, 30 Cell 933-943 (October 1982).

The pY α fGM-2 expression plasmid is transformed into an appropriate strain of Saccharomyces Cerevisiae (S. cerevisiae). Preferable strains include yeast strains Nos. 79, X2181-1B, DBY746, YNN282, 20B-12. These strains are all α, Trp 1 for compatibility with the α -factor promoter and for selection of Trp⁺ transformants. These strains are all widely available, for instance strain 79 is available from the Yeast Genetic Stock Center, Department of BioPhysics and Medical Physics, University of California, Berkeley, California 94702. The culture supernatants are assayed for biological activity through their ability to direct the formation of mixed, granulocytic and macrophage-type colonies from human bone marrow cells. As a control, plasmid pY α f, of the same construction as pY α fGM-2 but lacking the GM-CSF sequences was also transformed into a yeast host and the culture supernatant tested for biological activity. The pY α fGM-2 supernatant was found to direct synthesis of high levels of GM-CSF activity in the bone marrow colony assay ($1.25 \times 10^6$ CFU-C/ml) whereas no activity was detected from the supernatant derived from the pY α f control plasmid.

Analysis of mRNA

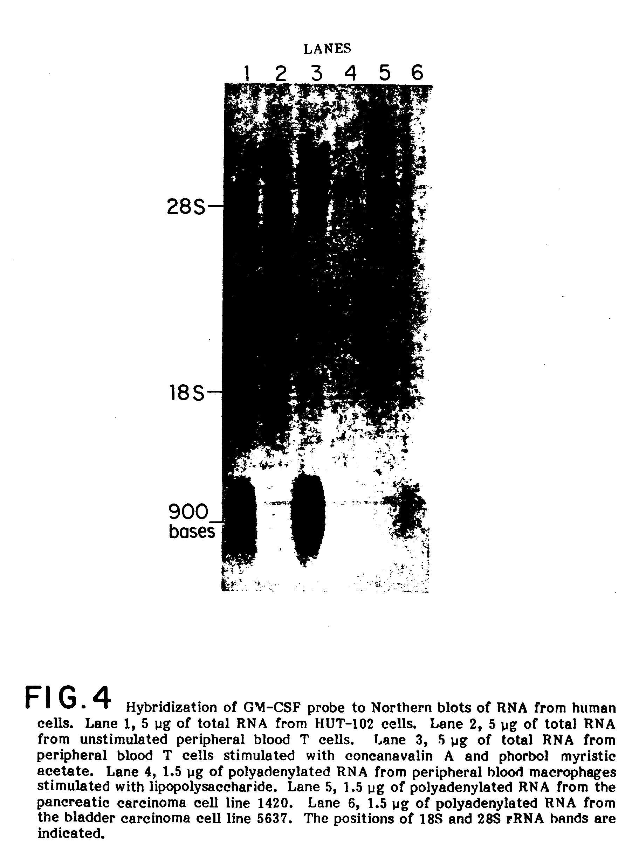

Since the availability of cells that synthesize GM-CSF is limited, the expression of GM-CSF mRNA from a number of cell types which have been reported previously to express other CSF was investigated. Northern blots of RNA from these cells were analyzed by hybridization with an RNA probe derived from pHG23. As shown in FIGURE 4, the probe is strongly hybridized to single bands of RNA derived from peripheral blood T-cells activated with PMA and Con A and from HUT-102 cells (lanes 3 and 1). A low level of hybridization occurred in RNA from a human bladder tumor cell line (lane 6). Also, no hybridization occurred in RNA from unstimulated T-cells, lipopolysaccharide-stimulated macrophages and a human pancreatic tumor cell line (lane 2, 4 and 5). These results are consistant with the level of biological activity found to be associated with GM-CSF derived from activated peripheral blood T-cells and from HUT-102 cells.

Analysis of Human Genomic Sequences

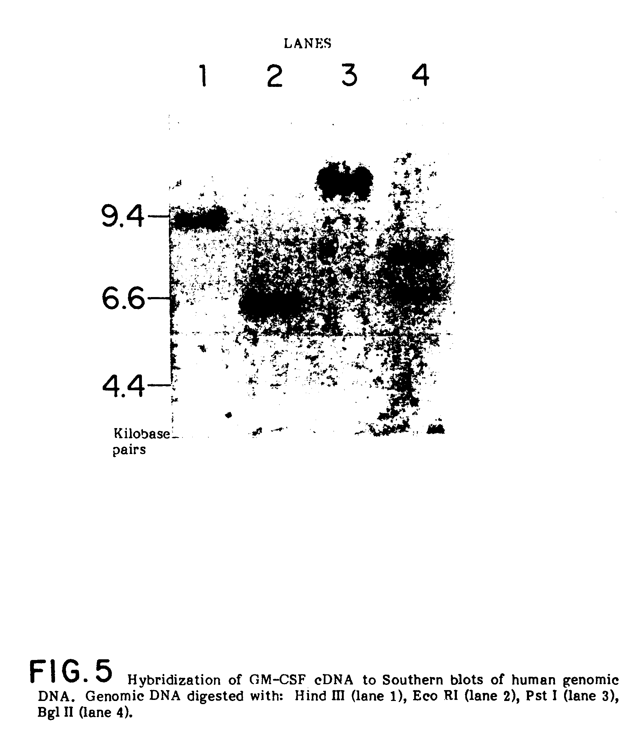

The number of GM-CSF related genes in human genomic DNA was investigated by hybridizing a $^{32}$P-labeled human GM-CSF probe to Southern blots of human genomic DNA fragments. The fragments were prepared by digesting human genomic DNA with a number of different restriction enzymes expected to cut the DNA relatively infrequently. As shown in FIGURE 5, digestion of the human genomic DNA with Hind III, Eco RI, or Pst I resulted in single bands, whereas digestion with Bgl II resulted in two hybridization bands. Based on these results, it appears that GM-CSF exists as a single copy gene in human genomic DNA.

The processes and products of the present invention are further illustrated by the following examples.

**EXAMPLE 1**

Preparation of Polyadenylated mRNA From Lymphoma T-Cells

HUT-102 cells at a concentration of approximately $2 \times 10^6$ cells per ml were cultured in 100-500 ml volumes in Roswell Park Memorial Institute ("RP MI")-1640 medium supplemented with 10% (v/v) fetal calf serum ("FCS"), 2 mM glutamine, 100 U/ml penicillin and 100 micrograms per milliliter ("ug/ml") streptomycin. The cells were cultured for approximately 3-5 days in a humidified atmosphere of 5% $CO_2$ in air. After this period of time, viable cells were harvested by centrifugation.

Total RNA was extracted from the HUT-102 cells by the standard method described by Chirgwin et al., supra. In this procedure guanidinium thiocyanate was used to denature the cellular protein including the RNase at a rate that exceeds the rate of RNA hydrolysis by RNase. The mRNA was removed from the cellular protein by ultracentrifugation through a dense cushion of cesium chloride.

Thereafter, polyadenylated mRNA was separated from the extracted protein on an oligo (dT)-cellulose chromatography column using the standard method disclosed by Maniatis et al., supra at 197. Briefly, the column was prepared with application buffer (20 mM Tris-Cl (pH 7.6), 0.5 M NaCl, 1 mM ethylene diamine tetra acetate ("EDTA") and 0.1% sodium dodecyl sulfate ("SDS")). The protein pellet was dissolved in water and application buffer and then loaded onto the column. The nonadsorbed material was removed from the column by initial washings with application buffer followed by additional washings with application buffer containing 0.1 M NaCl. The retained polyadenylated mRNA was eluted with buffers of reduced ionic strength composed of 10 mM Tris-Cl (pH 7.5), 1 mM EDTA and 0.05% SDS. The eluted polyadenylated mRNA was precipitated at -20°C with 1/10 volume sodium acetate (3M, pH 5.2) and 2.2 volumes of ethanol. After elution of the polyadenylated mRNA from the oligo (dT)-cellulose column, the integrity of the polyadenylated mRNA was confirmed by electrophoresis through agarose gels, by the standard method set forth in Maniatis et al., supra at 199.

**EXAMPLE 1A**

Preparation of Polyadenylated mRNA From Peripheral Blood T-Lymphoma Cells

Peripheral blood T-lymphocyte cells (mixture from Portland, Oregon Red Cross) at a concentration of approximately $2 \times 10^6$ cells per ml were cultured in 100-500 ml volumes in RP MI-1640 medium supplemented with 10% (v/v) FCS, 2 mM glutamine, 100 U/ml penicillin and 100 ug/ml streptomycin, together with 20 ug/ml concanavalin A ("Con A") (Pharmacia Fine Chemicals, Piscataway, NJ) and 10 ug/ml phorbal myristate acetate ("PMA") (Sigma Chemical Co., St. Louis, MO). The cells were cultured for approximately 20 hours in a humidified atmosphere of 5% $CO_2$ in air. After this period of time, viable cells were harvested by centrifugation. Thereafter, total RNA was extracted from the peripheral blood T-cells and polyadenylated mRNA prepared from the extracted protein as described in Example 1, supra.

**EXAMPLE 2**

Construction of cDNA Library

A library of double-stranded cDNA corresponding to the mRNA was prepared from the purified total mRNA in Examples 1 and 1A by employing the standard procedure detailed by Maniatis et al., supra at 229. Oligo-dT was hybridized to the polyadenylated tail of the mRNA to serve as the primer for the reverse transcription of the first cDNA strand. The enzyme avian myeloblastosis virus ("AMV") reverse transcriptase synthesized the first DNA strand by using the mRNA as a template. This procedure resulted in a hairpin loop being formed at the 3' end of the initial cDNA strand that serves as an integral primer for the second cDNA strand. After the mRNA strand had been degraded with NaOH, the second cDNA strand was synthesized with DNA polymerase I. The hairpin was then removed with nuclease S1 to produce double-stranded cDNA molecules.

The double-stranded cDNA was fractionated into size classes by Sephacryl S-400 (Pharmacia Fine Chemicals, Piscataway, N.J.) column chromatography and monitored by analysis using alkaline agarose electrophoresis employing end-labeled fragments of pBR322 DNA as molecular-weight markers. cDNA having a length of less than 500 bp was discarded to avoid needless cloning of these undersized cDNA fractions.

The double-stranded cDNA fractions, as prepared above, were inserted into the Pst I site of the pBR322 plasmid by the standard method contained in Maniatis et al., supra, beginning at 239. In this procedure, the double-stranded cDNA was tailed with poly (dC) at its 3' ends. The plasmid pBR322 (Pharmacia Fine Chemicals) was digested with Pst I endonuclease and then tailed with poly (dG) at its 3' ends. The tailed plasmid DNA and the tailed cDNA were annealed in annealing buffer (0.1M NaCl, 10 mM Tris-Cl (pH 7.8) and 10 mM ETDA) to form recombinant plasmids. All restriction enzymes described herein are commercially available from New England Biolabs, Beverly, Massachusetts.

The recombinant plasmids were transformed into E. coli strain MM294 by using the standard procedure of Hanahan, supra, in which the E. coli cells were prepared by growth in elevated levels of $Mg^{2+}$. The transformation hosts were plated and then transformants were identified by use of tetracycline as a phenotypic identifier. By this technique, applicants obtained approximately $2 \times 10^6$ independent transformants.

**EXAMPLE 3**

Preparation of Murine GM-CSF cDNA Screening Probe

Total RNA was extracted from LBRM-33-5A4 cells and polyadenlylated mRNA was separated therefrom on an oligo (dT)-cellulose chromatography column, using the protocols set forth in Examples 1 and 1A. The LBRM-33-5A4 cell line is available from the American Type Culture Collection, 12301 Park Lawn Drive, Rockville, MD 20852, U.S.A., No. ATCC-CRL-8080. The integrity of the resulting polyadenylated mRNA was confirmed by agarose gel electrophoresis. A library of double-stranded cDNA corresponding to the murine mRNA was prepared by the method set forth above in Example 2. The resulting double-stranded cDNA fractions of sizes greater than 500 bp were inserted into the Pst I site of the pBR322 plasmid by the homopolymeric tailing method set forth in Example 2. The recombinant plasmids were transformed into E. coli strain MM294 and then the transformants were identified by use of tetracycline as a phenotypic identifier. By this process, applicants identified approximately $6 \times 10^4$ independent transformants.

A synthetic oligonucleotide probe was chemically synthesized by standard triester method, as detailed by Sood et al., supra, and Hirose et al., supra, and then radiolabeled with $^{32}P$ for use in screening the murine cDNA library. The probe was composed of the following nucleotide sequence: 5'-TGATG GCCTCTACATGCTTCCAAGGCCGGGTAACAATTAT-3'. To facilitate labeling, the 5' ends of the oligonucleotides are synthesized with OH termini, thereby eliminating the phosphatase treatment which typically must be employed when labeling DNA fragments. The labeling protocol included adding one microliter ("ul") of the synthetic oligonueleotides to 16 ul of $^{32}P$-ATP (7000 Ci/mM), 1 ul (10 U) of T4 polynueleotide kinase and 2 ul of 10 x kinase buffer I(0.5 M Tris-Cl (pH 7.6), 0.1 $MgCl_2$, 50 mM dithiothreitol, 1 mM spermidine and 1 mM ETDA). The reaction was carried out at 37° C for thirty minutes, and thereafter the synthesized oligonucleotides were extracted with phenol/chloroform. The labeled probes were separated from unlabeled oligonucleotides by chromatography on Sephadex G-50 columns (Pharmacia Fine Chemicals).

To facilitate initial screening of the murine cDNA library, the transformed bacterial cultures were grouped into pools, each having approximately 3000 different clones. Plasmid DNA was removed from samples of the host bacteria by standard alkaline lysis method detailed by Ish-Horowicz and Burke, 9 Nucl. Acids Res. 2989 (1981). The isolated plasmids were digested to completion with Pvu II and Hind III by standard procedures. Next, the plasmid digests were fractionated by electrophoresis through 0.8% agarose gel and then blotted onto nitrocellulose filter by the standard method of Southern, supra. The DNA that bound to the nitrocellulose filter was hybridized with the labeled synthetic oligonucleotide probe using the procedure detailed in Example 4, infra. The putative pool(s) of clones from which hybridizing bands of DNA were obtained was screened by direct colony hybridization with the radiolabeled synthetic probe, and a single positive colony was identified.

Plasmid DNA was prepared from the identified positive colony by the procedures set forth above and then sequenced as discussed in Example 5, infra. The nucleotide sequence of the isolated murine plasmid DNA fragment, as shown in FIGURE 1, was found to include substantially the nucleotide sequence of the reading frame of murine GM-CSF gene plus additional nucleotides at the 5' terminus of the gene and one additional codon at an intermediate location along the coding region of the gene (nucleotide Nos. 201-203), thus confirming that the purified murine plasmid DNA codes for GM-CSF. As shown in FIGURE 1, several additional nonsignificant differences existed in the third nucleotides of several codons, which differences did not cause a change in the encoded amino acid.

The 356 bp fragment of the murine GM-CSF cDNA clone defined by the solid underline in FIGURE 1 (nucleotide No. 45 to nucleotide No. 400) was selected as a probe for screening the human plasmid DNA prepared in Example 2 above. The probe fragment was removed from the murine cDNA clone by double digestion with the restriction enzymes Pst I and Hae III followed by agarose gel electrophoresis.

The cDNA nucleotide probe was radiolabeled by nick translation by the standard procedure set forth in Maniatis et al., supra at 108 and discussed above at page 9. By this procedure, the probe was labeled to a specific activity of approximately 5 x $10^8$ CPM/ug DNA. Prior to use in screening protocols, the labeled probe was denatured by boiling in water at 100° C for ten minutes followed by chilling on ice.

## EXAMPLE 4

### Screening of cDNA Library

To facilitate initial screening of the cDNA library prepared in Example 2 above, the transformed bacteria cultures were grouped into pools each having approximately 100,000 different clones. Plasmid DNA was removed from samples of the host bacteria by standard alkaline lysis method detailed by Ish-Horowicz and Burke, 9 Nucl. Acids Res. 2989 (1981). The isolated plasmids were cleaved with Pst I and then fractionated by electrophoresis through 1.0% agarose gel with markers of appropriate size. The agarose gel was blotted onto nitrocellulose filter using the method described by Southern, supra. After the transfer process, the filter was air dried and baked for two hours at approximately 80° C under a vacuum to bind the DNA fragments to the nitrocellulose.

The bound DNA was next hybridized with the labeled cDNA probe. Briefly, the baked nitrocellulose was incubated at 55° C for 2-4 hours in prehybridization buffer composed of 6 x SSC, 0.5% NP40 detergent, 0.1% sarcosyl, 5 x Denhardt's solution (0.02% Ficoll, 0.02% polyvinyl pyrrolidone, 0.02% BSA) and 100 ug/ml denatured salmon sperin DNA (Sigma Type III, sodium salt). The filter was then incubated overnight at 55° C with the $^{32}$P-labeled cDNA probe ($10^6$ cpm/ml) (from Example 3) in hybridizing solution as above. After overnight hybridization, the filter was washed extensively with 6 x SSC at room temperature and then for 1 hour at 42° C and then for 1.5 hours at 55° C with 6 x SSC. After air drying, the filter was subjected to autoradiography at - 70° C.

From the autoradiography, applicants found a number of strongly hybridizing bands. One putative pool of clones from which the plasmid DNA that produced a strongly hybridizing band was obtained was subdivided into pools of approximately 7,000 transformants and the hybridization screening procedure repeated. The putative subpool from which a strongly hybridizing band of DNA was seen was then plated. The resulting colonies were probed with the radiolabeled cDNA nucleotide probe by the well-known methods of Grunstein and Hogness, supra, using the hybridizing conditions described above. By this process, a single positive host colony was identified.

## EXAMPLE 5

Characterization of Screened cDNA

Plasmid, designated as pHG23, was prepared with cDNA from the identified positive colony by the procedures set forth in Example 4. Samples of the host plasmid transformed into E. coli are on deposit with the ATCC under Accession No. 39900. The cDNA inserts prepared from the plasmid DNA removed from the positive host colony was sequenced by standard chain-termination protocol essentially as described in the Amersham Handbook, supra, with the variations set forth below. The cDNA insert was digested wish Pst I and/or Rsa I and then subcloned into strains mp18 and mp19 of the M13 single-stranded filamentous phage vector (Amersham, Arlington Heights, IL). The mp18 and mp19 phage vectors, as set forth in Norrander et al., supra, contain the following unique cloning sites: Hind III; Sph I; Pst I; Sal I; Acc I; Hinc II; Xba I; BamHI; Xma I; Sma I; Kpn I; Sst I; and, EcoRI. The composition of the mp18 and mp19 vectors are identical, with the exception that the order of the above-identified restriction sites are reversed in the mp19 vector so that both strands of the cDNA insert may be conveniently sequenced with the two vectors. The mp18 and mp19 vectors, with a corresponding strand of the cDNA inserted therein, were used to transform E. coli JM107 of the strain K12 (Bethesda Research Laboratories, Bethesda, MD) to produce relicate single-stranded DNA templates containing single-stranded inserts of the sense and antisense strands.

The synthetic universal primer: 5'-CCCAGTCACGACGTT-3' (P-L Biochemicals, Milwaukee, WI), was annealed to the single-strand DNA templates and used to prime DNA synthesis as described above at page 10. Thereafter, the extension fragments were size-separated by gel electrophoresis and auto-radiographed from which the nucleotide sequences of the fragments were deduced.

An additional primer of the composition 5'-GGCTGGCCATCATGGTC-3 was employed to prime synthesis from an intermediate location along the sense strands of the cDNA clones. The composition of the additional primer strand was established by sequencing of the cDNA subclones using the universal primer. By the above "walk down" method, the strands of the cDNA clones were sequenced in an overlapping, redundant manner thereby confirming their nucleotide sequences. It is to be understood that other synthetic primers could have been employed to initiate chain extensions from other locations along the strands of the cDNA clone, without departing from the scope of the present intention.

Deoxyadenosine 5' (alpha-[$^{35}$S] thio) triphosphate (hereinafter "dATP [alpha-$^{35}$S]") was used as the radioactive label in the dideoxy sequencing reactions. Also, rather than using the gel set forth at page 36 of the Amersham Handbook, a 6% polyacrylamide gel was employed (6% polyacrylmide gel, 0.4 mm thick, containing 7 M, urea 100 mM Tris borate (pH 8.1), and 2 mM EDTA).

As noted above, the nucleotide sequence of the cDNA is illustrated in FIGURE 2. The coding region of the human GM-CSF gene extends from nucleotide No. 14 (Ala residue) to nucleotide No. 394 (Glu residue), as shown in FIGURE 2. The corresponding amino acids, as determined by the nucleotide sequence, are set forth below the codons.

## EXAMPLE 6

Expression of Mature GM-CSF

Substantially the entire coding region and a portion of the 3' flanking region of the GM-CSF gene was removed from the cDNA clone of FIGURE 2 and employed to form a recombinant expression plasmid, designated as pY α fGM-2 to direct GM-CSF expression in yeast host cells. The pY α fGM-2 expression plasmid is on deposit with the ATCC under Assession No. 53157. As shown in FIGURE 3, pY α fGM-2 includes an origin of replication and an Ap$^r$ resistant gene from plasmid pBR322 (thick line portion). The expression plasmid also includes the yeast 2u circle origin of replication and a Trp I gene for selection of transformed yeast host (Trp - [Trp-auxotrophs], thin line portion in FIGURE 3). The expression plasmid further includes the α -factor promoter and leader sequences used to direct transcription and secretion of GM-CSF (solid box portion). The GM-CSF sequences (hatched box portion) are fused to the α -factor sequences with a synthetic oligonucleotide as discussed more fully below.

Substantially the entire coding region of the GM-CSF gene, from the Sfa NI to the Nco I site, was removed from the pHG23 clones by use of Sfa NI and Nco I restriction enzymes in a standard protocol, for instance as set forth in Maniatis et al., supra at 104. The GM-CSF gene segment was cleaved from the pHG23 clone at the Sfa NI site, which is located two nucleotides down stream from the 5' terminus of the region coding for the mature protein (nucleotide No. 14), since no restriction site was found to correspond precisely to nucleotide No. 14. An oligonucleotide was chemically synthesized to add back the 5' terminal portion of the coding region of the mature GM-CSF gene and also to add a second α -factor processing site to obtain complete processing of the signal for secretion of the mature form of GM-CSF. The composition of the oligonucleotide, as shown in Table I below, and in FIGURE 3, includes a Hind III cohesive 5' terminal, followed by a cathepsin B-like maturation site composed of the sequence: TCT TTG GAT AAA AGA, and a Sfa NI cohesive 3' terminus coding for the first two amino acid residues of the mature GM-CSF protein. Although the oligonucleotide shown in Table I is chemically synthesized by triester technique as detailed by Sood et al., supra and Hirose et al., supra, it is to be understood that the oligonucleotide can be prepared by other methods, such as by the phosphodiester method.

## TABLE 1

| 5' | A | GCT | TCT | TTG | GAT | AAA | AGA | GC | | 3' |
|---|---|---|---|---|---|---|---|---|---|---|
| | | | AGA | AAC | CTA | TTT | TCT | CGT | GGG | |
| | | | Ser | Leu | Asp | Lys | Arg | Ala | Pro | |

It is to be understood that other standard recombinant DNA techniques could be used to generate the same expression vector, and that the construction detailed above is representative of various strategies that could be used to prepare a GM-CSF cDNA fragment for insertion into the pY α fGM-2 expression vector.

The pY α fGM-2 was transformed into yeast strain 79 (α, Trp 1-1, Leu 2-1) of S. cerevisiae for selection of Trp$^+$ transformants by standard techniques. Prior to transformation, the strain 79 was grown in culture in YP-glucose medium to a density of $2 \times 10^7$ cells/ml. Cells were harvested by centrifugation at 1000 x g for 5 minutes at 22°C, and then the resulting pellet was washed with sterile, distilled water.

The yeast cells were then concentrated by resuspending in 1/10 vol. of SED (1 M sorbitol, 25 mM EDTA [pH 8.0], and 50 mM dithiothreitol) and incubating for 10 minutes at 30°C. The cell-buffer mixture was then centrifuged for 5 minutes at 300 x g. The pellet was washed once with 1/10 vol. of 1 M sorbitol and the cells resuspended in 20 milliliters of SCE (1 M sorbitol, 0.1 M sodium citrate [pH 5.8], 0.01 M EDTA). Glusulase, to break down the cell walls, in an amount of $10^{-3}$ vol. was added to the solution and then the solution incubated at 30°C for 30 minutes with occasional gentle shaking. The presence of spheroplasts was assayed by diluting 10 microliters of the yeast cells into a drop of 5% SDS (wt./vol.) on a microscope slide to observe for "ghosts" at 400 X phase contrast. The cell mixture was then centrifuged at 300 x g for 3 minutes. The resulting pellet was twice washed with 1/10 vol. of 1 M sorbitol. The pellet was then once washed in CaS (1 M sorbitol, 10 mM CaCl$_2$).

The yeast spheroplasts were then transformed with the previously prepared plasmid vector in a procedure adapted from Beggs, supra. The pelleted spheroplasts were suspended in 1/200 vol. of CaS and then divided into 100 microliter aliquotes in 1.5 ml Eppendorf tubes. Then, from 1 to 10 ul of the plasmid DNA were added to each aliquot (0.5 to 5 ug). The mixture was incubated at room temperature for 10 minutes and then 1 ml of PEG (20% PEG 4000, 10 mM CaCl$_2$, 10 mM Tris-HCl [pH 7.4]) was added to each aliquot to promote DNA uptake. After 10 minutes at room temperature, the mixture was centrifuged for 5 minutes at 350 x g. The resulting pellet was resuspended in 150 ul of SOS (10 ml of 2 M sorbitol, 6.7 ml of YEP [1% (wt/vol) yeast extract, 2% (wt/vol) peptone, 2% (wt/vol) glucose], 0.13 ml of 1 M CaCl$_2$, 27 ul of 1% tryptophane and 3.7 ml of water). This mixture was incubated for 20 minutes at 30°C. The cells were then plated, or held at 4°C for up to a few days.

Prior to plating the protoplast/DNA mixture selective plates were preincubated at 37°C. Three ml of melted top agar (45°C), composed of 18.2 ml of sorbitol, 2 gm agar, 0.6 gm Difco yeast nitrogen base (without amino acids), 2 gm glucose, 0.1 ml of 1% adenine, 0.4 ml of 1% uracil and amino acids as required, was then added to each aliquot of transformed cells and the tube contents poured on the selective plates. The plates were incubated from 2 to 4 days at 30°C. Colonies which developed in the Trp minus medium contained plasmids that have the Trp 1 gene, i.e., those that are transformed.

Prior to biological assay, the transformants were grown in 20-50 ml of rich medium (1% yeast extract, 2% peptone, 2% glucose) at 33°C to stationary phase. At the time of harvest, the protase inhibitors phenyl methyl sulfonyl (P MSF) and Pepstatin A were added to a final concentration of 1 mM and 10 uM, respectively. The cells were then removed by centrifugation at 400 x g and the medium was filtered through a 0.45 u cellulose acetate filter.

**EXAMPLE 7**

Colony Assay

The presence of human GM-CSF harvested from the yeast cultures in Example 6 was confirmed by assaying the ability of the supernatant to stimulate growth of human bone marrow colonies in agar. For use in the assay, human bone marrow from the iliac crest of healthy donors was collected in a heparinized syringe. The marrow was diluted 1:3 with phosphate buffered saline (PBS) at room temperature and layered onto a solution of 54% percoll (Pharmacia Fine Chemicals). After centrifugation at 500 x g at room temperature for 20 minutes, the interface was collected and washed with 20 volumes of PBS. The suspension was then centrifuged at 250 x g for 10 minutes at room temperature. The cells were then resuspended in 10 ml of $\alpha$ -Minimal Essential Medium with nucleotides ($\alpha$ -Mem, Gibco) for cell counting and viability determination. FCS was then added and the cell suspension stored on ice until the assay was carried out.

In the assay, bone marrow cells as prepared above were added at a final concentration of 1 x $10^5$/ml to an incubation medium consisting of: (a) seven parts of a solution containing 28.1% FCS, 0.7 x $10^{-4}$ M 2-Mercaptoethanol, 0.12 mg/ml asparagine, 0.7 mg/ml glutamine, 150 units of penicillin G, 150 units of streptomycin, 1.1 x $\alpha$-MEM with nucleotides, and 2.2 x vitamins (Gibco); and, (b) three parts of 1.4% bacto-agar solution (Difco). The cultures were incubated in a humidified atmosphere at 37° C in the presence of 5% $CO_2$. After seven to fourteen days of culture, the number and types of colonies, whether granulocyte, macrophage or mixed granulocyte-macrophage, were determined. Applicants found that the GM-CSF gene from the pY $\alpha$ fGM-2 clones directed synthesis of GM-CSF activity at the high level of 1.25 x $10^6$ colony forming units ("CFU") per milliliter. This activity level was determined by multiplying by 50 the reciprocal of the dilution giving 50% of the maximum colony number. Applicants have found that the average number of colonies from 1 x $10^5$ bone marrow cells was 96 ± 29. The colonies formed at 14 days by the recombinant GM-CSF were well defined and consisted of three types: approximately 1/3 mixed granulocyte-macrophage colonies; approximately 1/3 tight granulocyte colonies, and approximately 1/3 dispersed macrophage colonies.

As a further control for the expression system of the present invention, a plasmid identical to pY $\alpha$ fGM-2, but lacking the GM-CSF sequences, was also transformed into yeast strain 79. The culture supernatant from the yeast produced no GM-CSF activity in the bone marrow colony assay.

As a positive control, supernatants from human placental cells, a natural source of GM-CSF, were also tested for colony forming activity. The human placental cells were cultured at 1.2 x $10^7$/ml for 6 days in the presence of 5% fetal bovine serum. When tested in the bone marrow assay, the supernate from the cultured placenta cells were found to have an activity of approximately 5 x $10^2$ CFU-C/ml, and to give approximately the same relative levels of each colony type: 1/3 mixed granulocyte-macrophage colonies, 1/3 tight granulocyte colonies and 1/3 dispersed macrophage colonies.

## EXAMPLE 8

Analysis of mRNA

The expression of GM-CSF mRNA from a number of cell types, which have been reported previously to express other CSF, was investigated. Northern blots of RNA from these cells were analyzed by hybridization with a probe derived from pHG23. In this regard, total RNA for Northern blots was isolated by the guanidium thiocyanate/cesium chloride method as set forth supra in Example 1, from the following cells: (1) Hut-102 cells; (2) unstimulated peripheral blood T-cells; (3) peripheral blood T-cells stimulated with Con A and PMA as described supra in Example 1A; (4) peripheral blood macrophages stimulated with lipopolysaccharide; (5) pancreatic carcinoma cell line 1420 (from ATCC); and, (6) bladder carcinoma cell line 5637 (from ATCC). The RNA samples were sized by electrophoresis in 1.1% agarose gels containing formaldehyde to denature the RNA so that the rate of migration of the RNA through the gel was in proportional to its molecular weight. A standard protocol for electrophoresis of the RNA through agarose gels containing formaldehyde is set forth in Maniatis et al., supra at 202.

After electrophoresis the formaldehyde-denatured RNA was transferred to nitrocellulose filters using a standard protocol as detailed in Maniatis et al., supra at 203, for subsequent hybridization with $^{32}$P-labeled RNA probe transcribed In vitro by SP6 polymerase by the procedure set forth in Green et al., 32 Cell 681 (March 1981). The $^{32}$P-RNA probe was synthesized from the 600 base pair Pst I to Nco I fragment of pHG23 which was subcloned into the pSP64 vector (Promega Biotech). The RNA bound to the nitrocellulose filters was hybridized with the labeled RNA probe ($10^6$ cpm/ml) for 16 hours at 63° C in Stark's complete buffer: 5 x SSC; 50 mM $KH_2$ $PO_4$ [pH 2.5]; 150ug/ml denatured salmon sperm DNA; 2 x Denhardt's solution [0.04% (wt/vol) Ficoll, 0.04% (wt/vol) polyvinyl pyrrolidone, 0.04% (wt/vol) BSA]; 0.1% SDS; 20 mM Na$_2$ EDTA; and, 50% (wt/vol) formamide. After hybridization, the filter was washed at 63° in 6 x SSC for two hours and in 0.1 x SSC for two hours and then autoradiographed for four hours with intensifying screens at -70°.

The results of the autoradiography are set forth in FIGURE 4 which shows strong hybridization to a band of approximately 900 nucleotides in 5 ug of total RNA from PMA and Con A activated peripheral blood T-cells and from Hut 102 cells (lanes 3 and 4). A low level of hybridization was seen in 1.5 ug of polyadenylated RNA from the human blood carcinoma cell line 5637 (lane 6). No hybridization was seen in 5 ug of total RNA from unstimulated peripheral blood T-cells (lane 2), 1.5 ug of polyadenylated RNA from lipopolysaccharide-stimulated peripheral blood macrophages (lane 4) and 1.5 ug of polyadenylated RNA from human pancreatic carcinoma cell line 1420 (lane 5). The other high molecular weight bands shown in FIGURE 4, 18S and 28S, are due to the hybridization of the probe to ribosomal RNA. The results from the Northern blot analysis are consistent with the high level of biological activity found to be associated with GM-CSF derived from activated peripheral blood T-cells and HUT-102 cells.

## EXAMPLE 9

### Analysis of Human Genomic Sequences

To determine the number of GM-CSF-related genes in human genomic DNA, a labeled GM-CSF cDNA probe was hybridized to Southern blots of human DNA digested with restriction enzymes expected to cut relatively infrequently. The probe included the entire pHG23 sequence (Figure 2) 3' of nucleotide number 161 and was radiolabeled by nick translation by the standard procedure set forth in Maniatis et al., supra at 108 and discussed at page 10 herein. Prior to hybridization, 10 ug of human genomic DNA was digested to completion with Hind III, Eco RI, Pst I, or Bgl II using standard techniques. The digested human DNA was fractionated by electrophoresis in a 0.7% agarose gel with markers of appropriate size. The agarose gel was blotted onto nitrocellulose filters using the method described by Southern, supra. After the transfer process the filter was air-dried and baked for two hours at 80°C to bind the DNA fragments to the nitrocellulose. Thereafter, the bound DNA was hybridized with the labeled cDNA probe as set forth in Example 4, supra, then washed extensively in 2 x SSC, 0.5% SDS at room temperature followed by washing in 0.1 x SSC, 0.5% SDS for 45 minutes at 65°C. After air drying, the filter was subjected to autoradiography at -70°C.

The results of autoradiography are set forth in FIGURE 5. In FIGURE 5 the molecular weight markers (in kilobase pairs) are from Hind III-digested bacteriophage λ DNA. As shown in FIGURE 5, human DNA digested with Hind III, Eco RI, and Pst I (lanes 1, 2, 3, respectively) resulted in single bands, while digestion with Bgl II gave rise to two bands. On this basis, it appears that GM-CSF exists as a single copy gene in human genomic DNA.

### Claims
### Claims for the following Contracting States: BE, FR, DE, IT, LU, NL, SE, CH, LI, GB

1. A recombinant DNA expression vector comprising a promoter that directs expression in a yeast host of a recombinant DNA coding sequence which:

   (a) encodes a mature human granulocyte macrophage colony stimulating factor (GM-CSF) protein having N-terminal alanine-proline residues, which protein is capable of stimulating growth of human bone marrow colonies in the human bone marrow colony assay;
   (b) hybridizes to a radiolabeled single-stranded DNA probe consisting of a PstI-Hae III fragment of the murine GM-CSF gene corresponding to nucleotides 45 through 400 indicated in Figure 1, after overnight hybridization in 6xSSC at 55°C followed by washing with 6xSSC; and
   (c) is fused at its 5' terminus to a leader sequence derived from a yeast mating pheromone gene that directs secretion of said mature human GM-CSF protein into culture medium upon cleavage of such leader from said N-terminal alanine-proline residues.

2. A recombinant DNA expression vector according to claim 1, wherein the recombinant DNA coding sequence encodes a mature human GM-CSF protein having the amino acid sequence:

```
Ala   Pro   Ala   Arg   Ser   Pro   Ser   Pro   Ser   Thr
Gln   Pro   Trp   Glu   His   Val   Asn   Ala   Ile   Gln
Glu   Ala   Arg   Arg   Leu   Leu   Asn   Leu   Ser   Arg
Asp   Thr   Ala   Ala   Glu   Met   Asn   Glu   Thr   Val
Glu   Val   Ile   Ser   Glu   Met   Phe   Asp   Leu   Gln
Glu   Pro   Thr   Cys   Leu   Gln   Thr   Arg   Leu   Glu
Leu   Tyr   Lys   Gln   Gly   Leu   Arg   Gly   Ser   Leu
Thr   Lys   Leu   Lys   Gly   Pro   Leu   Thr   Met   Met
Ala   Ser   His   Tyr   Lys   Gln   His   Cys   Pro   Pro
Thr   Pro   Glu   Thr   Ser   Cys   Ala   Thr   Gln   Ile
Ile   Thr   Phe   Glu   Ser   Phe   Lys   Glu   Asn   Leu
Lys   Asp   Phe   Leu   Leu   Val   Ile   Pro   Phe   Asp
Cys   Trp   Glu   Pro   Val   Gln   Glu.
```

3. A recombinant DNA expression vector according to claim 2, wherein the recombinant DNA coding sequence consists of the nucleotide sequence:

```
GCA   CCC   GCC   CGC   TCG   CCC   AGC   CCC   AGC   ACA
CAG   CCC   TGG   GAA   CAT   GTG   AAT   GCC   ATC   CAG
GAA   GCC   CGG   CGT   CTC   CTG   AAC   CTG   AGT   AGA
GAC   ACT   GCT   GCT   GAG   ATG   AAT   GAA   ACA   GTA
GAA   GTC   ATC   TCA   GAA   ATG   TTT   GAC   CTC   CAG
GAG   CCG   ACC   TGC   CTA   CAG   ACC   CGC   CTG   GAG
CTG   TAC   AAG   CAG   GGC   CTG   CGG   GGC   AGC   CTC
ACC   AAG   CTC   AAG   GGC   CCC   TTG   ACC   ATG   ATG
GCC   AGC   CAC   TAC   AAA   CAG   CAC   TGC   CCT   CCA
ACC   CCG   GAA   ACT   TCC   TGT   GCA   ACC   CAG   ATT
ATC   ACC   TTT   GAA   AGT   TTC   AAA   GAG   AAC   CTG
```

```
AAG   GAC   TTT   CTG   CTT   GTC   ATC   CCC   TTT   GAC
TGC   TGG   GAG   CCA   GTC   CAG   GAG.
```

4. A recombinant DNA expression vector according to any of claims 1 to 3, wherein the DNA encoding the human GM-CSF protein is linked to the yeast α-factor leader sequence.

5. The recombinant DNA expression vector pYαfGM-2 (ATCC 53157).

6. A yeast host cell transformed with a recombinant DNA expression vector according to any of claims 1-5.

7. A process for producing a mature human granulocyte-macrophage colony stimulating factor (GM-CSF) protein, comprising growing a yeast host according to claim 6 in a culture medium and recovering the human GM-CSF protein from the culture medium.

**Claims for the following Contracting State: AT**

1.  A process for the preparation of a recombinant DNA expression vector comprising a promoter that directs expression in a yeast host of a recombinant DNA coding sequence which:

    (a) encodes a mature human granulocyte macrophage colony stimulating factor (GM-CSF) protein having N-terminal alanine-proline residues, which protein is capable of stimulating growth of human bone marrow colonies in the human bone marrow colony assay;
    (b) hybridizes to a radiolabeled single-stranded DNA probe consisting of a PstI-Hae III fragment of the murine GM-CSF gene corresponding to nucleotides 45 through 400 indicated in Figure 1, after overnight hybridization in 6xSSC at 55ºC followed by washing with 6xSSC; and
    (c) is fused at its 5' terminus to a leader sequence derived from a yeast mating pheromone gene that directs secretion of said mature human GM-CSF protein into culture medium upon cleavage of such leader from said N-terminal alanine-proline residues;

    the process comprising coupling together successive nucleotides

2.  A process according to claim 1 wherein the recombinant DNA coding sequence encodes a mature human GM-CSF protein having the amino acid sequence:

| | | | | | | | | | |
|-----|-----|-----|-----|-----|-----|-----|-----|-----|-----|
| Ala | Pro | Ala | Arg | Ser | Pro | Ser | Pro | Ser | Thr |
| Gln | Pro | Trp | Glu | His | Val | Asn | Ala | Ile | Gln |
| Glu | Ala | Arg | Arg | Leu | Leu | Asn | Leu | Ser | Arg |
| Asp | Thr | Ala | Ala | Glu | Met | Asn | Glu | Thr | Val |
| Glu | Val | Ile | Ser | Glu | Met | Phe | Asp | Leu | Gln |
| Glu | Pro | Thr | Cys | Leu | Gln | Thr | Arg | Leu | Glu |
| Leu | Tyr | Lys | Gln | Gly | Leu | Arg | Gly | Ser | Leu |
| Thr | Lys | Leu | Lys | Gly | Pro | Leu | Thr | Met | Met |
| Ala | Ser | His | Tyr | Lys | Gln | His | Cys | Pro | Pro |
| Thr | Pro | Glu | Thr | Ser | Cys | Ala | Thr | Gln | Ile |
| Ile | Thr | Phe | Glu | Ser | Phe | Lys | Glu | Asn | Leu |
| Lys | Asp | Phe | Leu | Leu | Val | Ile | Pro | Phe | Asp |
| Cys | Trp | Glu | Pro | Val | Gln | Glu. | | | |

3.  A process according to claim 2, wherein the recombinant DNA coding sequence consists of the nucleotide sequence:

```
GCA   CCC   GCC   CGC   TCG   CCC   AGC   CCC   AGC   ACA

CAG   CCC   TGG   GAA   CAT   GTG   AAT   GCC   ATC   CAG

GAA   GCC   CGG   CGT   CTC   CTG   AAC   CTG   AGT   AGA

GAC   ACT   GCT   GCT   GAG   ATG   AAT   GAA   ACA   GTA

GAA   GTC   ATC   TCA   GAA   ATG   TTT   GAC   CTC   CAG

GAG   CCG   ACC   TGC   CTA   CAG   ACC   CGC   CTG   GAG

CTG   TAC   AAG   CAG   GGC   CTG   CGG   GGC   AGC   CTC

ACC   AAG   CTC   AAG   GGC   CCC   TTG   ACC   ATG   ATG

GCC   AGC   CAC   TAC   AAA   CAG   CAC   TGC   CCT   CCA



ACC   CCG   GAA   ACT   TCC   TGT   GCA   ACC   CAG   ATT

ATC   ACC   TTT   GAA   AGT   TTC   AAA   GAG   AAC   CTG

AAG   GAC   TTT   CTG   CTT   GTC   ATC   CCC   TTT   GAC

TGC   TGG   GAG   CCA   GTC   CAG   GAG.
```

**4.** A process according to any of claims 1 to 3, wherein the DNA encoding the human GM-CSF protein is linked to the yeast α-factor leader sequence.

**5.** A process for the preparation of the recombinant DNA expression vector pYαfGM-2 (ATCC 53157), the process comprising coupling together successive nucleotides.

**6.** A process for the preparation of a transformed yeast host cell, the process comprising transforming a yeast host cell with a recombinant DNA expression vector according to any of claims 1 to 5.

**7.** A process for producing a mature human granulocyte-macrophage colony stimulating factor (GM-CSF) protein, comprising growing a yeast host prepared by a process according to claim 6 in a culture medium and recovering the human GM-CSF protein from the culture medium.

**Revendications**

**Revendications pour les Etats contractants suivants: BE, FR, DE, IT, LU, NL, SE, CH, LI, GB**

**1.** Un vecteur d'expression d'ADN recombinant comprenant un promoteur qui dirige l'expression dans une cellule de levure hôte ou une séquence de codage d'ADN recombinant qui:

(a) code une protéine de facteur stimulant de colonies de macrophages de granulocytes (GM-CSF) humain mûr possédant des résidus N-terminaux alanine-proline, laquelle protéine est capable de stimuler la croissance de colonies de moelle osseuse humaine dans l'essai de colonie de moelle osseuse humaine;

(b) s'hybride avec un échantillon à un seul brin d'ADN radioactivé composé d'un fragment PstI-Hae III du gène GM-CSF murin correspondant aux nucléotides 45 à 400 indiqués dans la figure 1, après hybridation pendant une nuit entière dans du 6xSSC à 55°C suivie d'un lavage au 6xSSC; et

(c) est fusionnée en son terminal 5' avec une séquence de tête dérivée d'un gène de phéromone de maturation de la levure qui dirige la sécrétion de ladite protéine de GM-CSF humain mûr dans le milieu de culture par clivage de cette tête desdits résidus N-terminaux alanine-proline.

**2.** Un vecteur d'expression d'ADN recombinant selon la revendication 1 dans lequel la séquence de codage d'ADN recombinant code une protéine de GM-CSF humain mûr possédant la séquence d'acides aminés suivante:

```
Ala   Pro   Ala   Arg   Ser   Pro   Ser   Pro   Ser   Thr

Gln   Pro   Trp   Glu   His   Val   Asn   Ala   Ile   Gln

Glu   Ala   Arg   Arg   Leu   Leu   Asn   Leu   Ser   Arg

Asp   Thr   Ala   Ala   Glu   Met   Asn   Glu   Thr   Val

Glu   Val   Ile   Ser   Glu   Met   Phe   Asp   Leu   Gln

Glu   Pro   Thr   Cys   Leu   Gln   Thr   Arg   Leu   Glu

Leu   Tyr   Lys   Gln   Gly   Leu   Arg   Gly   Ser   Leu

Thr   Lys   Leu   Lys   Gly   Pro   Leu   Thr   Met   Met

Ala   Ser   His   Tyr   Lys   Gln   His   Cys   Pro   Pro

Thr   Pro   Glu   Thr   Ser   Cys   Ala   Thr   Gln   Ile

Ile   Thr   Phe   Glu   Ser   Phe   Lys   Glu   Asn   Leu

Lys   Asp   Phe   Leu   Leu   Val   Ile   Pro   Phe   Asp

Cys   Trp   Glu   Pro   Val   Gln   Glu.
```

3.  Un vecteur d'expression d'ADN recombinant selon la revendication 2 dans lequel la séquence de codage d'ADN recombinant se compose de la séquence de nucléotides suivante :

```
GCA   CCC   GCC   CGC   TCG   CCC   AGC   CCC   AGC   ACA

CAG   CCC   TGG   GAA   CAT   GTG   AAT   GCC   ATC   CAG

GAA   GCC   CGG   CGT   CTC   CTG   AAC   CTG   AGT   AGA

GAC   ACT   GCT   GCT   GAG   ATG   AAT   GAA   ACA   GTA

GAA   GTC   ATC   TCA   GAA   ATG   TTT   GAC   CTC   CAG

GAG   CCG   ACC   TGC   CTA   CAG   ACC   CGC   CTG   GAG

CTG   TAC   AAG   CAG   GGC   CTG   CGG   GGC   AGC   CTC

ACC   AAG   CTC   AAG   GGC   CCC   TTG   ACC   ATG   ATG

GCC   AGC   CAC   TAC   AAA   CAG   CAC   TGC   CCT   CCA

ACC   CCG   GAA   ACT   TCC   TGT   GCA   ACC   CAG   ATT

ATC   ACC   TTT   GAA   AGT   TTC   AAA   GAG   AAC   CTG

AAG   GAC   TTT   CTG   CTT   GTC   ATC   CCC   TTT   GAC

TGC   TGG   GAG   CCA   GTC   CAG   GAG
```

4.  Un vecteur d'expression d'ADN recombinant selon une des revendications 1 à 3 dans lequel l'ADN codant la protéine de GM-CSF humain est lié à la séquence de tête du facteur α de levure.

5.  Le vecteur d'expression d'ADN recombinant pYαfGM-2 (ATCC 53157).

6.  Une cellule hôte de levure transformée avec un vecteur d'expression d'ADN recombinant selon une des revendications 1 à 5.

**7.** Un procédé de préparation d'une protéine de facteur stimulant de colonies humain mûr de macrophages de granulocytes, comprenant la croissance d'une levure hôte selon la revendication 6 dans un milieu de culture et la récupération de la protéine de GM-CSF humain à partir du milieu de culture.

**Revendications pour l'Etat contractant suivant: AT**

**1.** Un procédé de préparation d'un vecteur d'expression d'ADN recombinant comprenant un promoteur qui dirige l'expression dans une levure hôte d'une séquence de codage d'ADN recombinant qui:

(a) code une protéine de facteur stimulant de colonies de macrophages de granulocytes humain mûr (GM-CSF) possédant des résidus N-terminaux alanine-proline, laquelle protéine est capable de stimuler la croissance de colonies de moelle osseuse humaine dans l'essai de colonie de moelle osseuse humaine;

(b) s'hybride avec un échantillon à un seul brin d'ADN radioactivé composé d'un fragment PstI-Hae III du gène GM-CSF murin correspondant aux nucléotides 45 à 400 indiqués dans la figure 1, après hybridation pendant une nuit entière dans du 6xSSC à 55°C suivie d'un lavage au 6xSSC; et

(c) est fusionnée en son terminal 5' avec une séquence de tête dérivée d'un gène de phéromone de maturation de la levure qui dirige la secrétion de ladite protéine de GM-CSF humain mûr dans le milieu de culture par clivage de cette tête à partir desdits résidus N-terminaux alanine-proline;

le procédé comprenant le couplage de nucléotides successifs.

**2.** Un procédé selon la revendication 1 dans lequel la séquence de codage d'ADN recombinant code une protéine de GM-CSF humain mûr possédant la séquence d'acides aminés suivante:

| | | | | | | | | | |
|---|---|---|---|---|---|---|---|---|---|
| Ala | Pro | Ala | Arg | Ser | Pro | Ser | Pro | Ser | Thr |
| Gln | Pro | Trp | Glu | His | Val | Asn | Ala | Ile | Gln |
| Glu | Ala | Arg | Arg | Leu | Leu | Asn | Leu | Ser | Arg |
| Asp | Thr | Ala | Ala | Glu | Met | Asn | Glu | Thr | Val |
| Glu | Val | Ile | Ser | Glu | Met | Phe | Asp | Leu | Gln |
| Glu | Pro | Thr | Cys | Leu | Gln | Thr | Arg | Leu | Glu |
| Leu | Tyr | Lys | Gln | Gly | Leu | Arg | Gly | Ser | Leu |
| Thr | Lys | Leu | Lys | Gly | Pro | Leu | Thr | Met | Met |
| Ala | Ser | His | Tyr | Lys | Gln | His | Cys | Pro | Pro |
| Thr | Pro | Glu | Thr | Ser | Cys | Ala | Thr | Gln | Ile |
| Ile | Thr | Phe | Glu | Ser | Phe | Lys | Glu | Asn | Leu |
| Lys | Asp | Phe | Leu | Leu | Val | Ile | Pro | Phe | Asp |
| Cys | Trp | Glu | Pro | Val | Gln | Glu. | | | |

**3.** Un procédé selon la revendication 2 dans lequel la séquence de codage d'ADN recombinant se compose de la séquence de nucléotides suivante:

```
GCA   CCC   GCC   CGC   TCG   CCC   AGC   CCC   AGC   ACA
CAG   CCC   TGG   GAA   CAT   GTG   AAT   GCC   ATC   CAG
GAA   GCC   CGG   CGT   CTC   CTG   AAC   CTG   AGT   AGA
GAC   ACT   GCT   GCT   GAG   ATG   AAT   GAA   ACA   GTA
GAA   GTC   ATC   TCA   GAA   ATG   TTT   GAC   CTC   CAG
GAG   CCG   ACC   TGC   CTA   CAG   ACC   CGC   CTG   GAG
CTG   TAC   AAG   CAG   GGC   CTG   CGG   GGC   AGC   CTC
ACC   AAG   CTC   AAG   GGC   CCC   TTG   ACC   ATG   ATG
GCC   AGC   CAC   TAC   AAA   CAG   CAC   TGC   CCT   CCA
ACC   CCG   GAA   ACT   TCC   TGT   GCA   ACC   CAG   ATT
ATC   ACC   TTT   GAA   AGT   TTC   AAA   GAG   AAC   CTG
AAG   GAC   TTT   CTG   CTT   GTC   ATC   CCC   TTT   GAC
TGC   TGG   GAG   CCA   GTC   CAG   GAG
```

4. Un procédé selon une des revendications 1 à 3 dans lequel l'ADN codant la protéine de GM-CSF humain est lié à la séquence de tête du facteur α de levure.

5. Un procédé de préparation du vecteur d'expression de l'ADN recombinant pYα fGM-2 (ATCC 53157), le procédé comprenant le couplage de nucléotides successifs.

6. Un procédé de préparation d'une cellule hôte de levure transformée, le procédé comprenant la transformation d'une cellule hôte de levure avec un vecteur d'expression d'ADN recombinant selon une des revendications 1 à 5.

7. Un procédé de préparation d'une protéine de facteur stimulant de colonies humain mûr de macrophages de granulocytes (GM-CSF), comprenant la croissance d'une levure hôte préparée par un procédé selon la revendication 6 dans un milieu de culture et la récupération de la protéine de GM-CSF humain à partir du milieu de culture.

**Patentansprüche**

**Patentansprüche für folgende Vertragsstaaten: BE, FR, DE, IT, LU, NL, SE, CH, LI, GB**

1. Expressionsvektor für die Expression von rekombinanter DNA, welcher einen Promotor enthält, der seinerseits in einer Hefewirtszelle die Expression einer Kodiersequenz von rekombinanter DNA steuert, welche Kodiersequenz:

   (a) für ein Protein des reifen, Human-Granulozyten-Makrophagen-Kolonien stimulierenden Faktors (GM-CSF) kodiert, welches Protein N-terminale Alanin-Prolin-Reste aufweist, und welches Protein befähigt ist, das Wachstem von Human-Knochenmarks-Kolonien in dem Human-Knochenmarks-Kolonien-Assay zu stimulieren;

   (b) sich an eine radioaktiv markierte, einzelsträngige DNA-Sonde hybridisiert, welche aus einem PstI-Hae-III-Fragment des Mäuse-GM-CSF-Gens besteht, welches Fragment den in der Fig. 1 dargestellten Nikleotiden 45 bis 400 entspricht, und zwar nach einer Über-Nacht-Hybridisierung in 6 x SSC bei 55° C und einem anschließenden Waschen mit 6 x SSC; und

   (c) an ihrem 5'-Terminus an eine Leitsequenz fusioniert ist, welche aus einem entsprechenden Hefe-Pheromon-Gen stammt, und welche die Abssonderung des reifen Human-GM-CSF-Proteins in das Kulturmedium nach Abspalten der Leitsequenz von den N-terminalen Alanin-Prolin-Resten steuert.

2. Expressionsvektor für die Expression von rekombinanter DNA nach Anspruch 1, worin die Kodiersequenz von rekombinanter DNA für ein Protein des reifen Human-GM-CSF kodiert, welches Protein die

Aminosäurensequenz:

```
Ala  Pro  Ala  Arg  Ser  Pro  Ser  Pro  Ser  Thr
Gln  Pro  Trp  Glu  His  Val  Asn  Ala  Ile  Gln
Glu  Ala  Arg  Arg  Leu  Leu  Asn  Leu  Ser  Arg
Asp  Thr  Ala  Ala  Glu  Met  Asn  Glu  Thr  Val
Glu  Val  Ile  Ser  Glu  Met  Phe  Asp  Leu  Gln
Glu  Pro  Thr  Cys  Leu  Gln  Thr  Arg  Leu  Glu
Leu  Tyr  Lys  Gln  Gly  Leu  Arg  Gly  Ser  Leu
Thr  Lys  Leu  Lys  Gly  Pro  Leu  Thr  Met  Met
Ala  Ser  His  Tyr  Lys  Gln  His  Cys  Pro  Pro
Thr  Pro  Glu  Thr  Ser  Cys  Ala  Thr  Gln  Ile
Ile  Thr  Phe  Glu  Ser  Phe  Lys  Glu  Asn  Leu
Lys  Asp  Phe  Leu  Leu  Val  Ile  Pro  Phe  Asp
Cys  Trp  Glu  Pro  Val  Gln  Glu.
aufweist.
```

3. Expressionsvektor für die Expression von rekombinanter DNA nach Anspruch 2, worin die Kodiersequenz von rekombinanter DNA aus der folgenden Nukleotidsequenz besteht:

```
GCA  CCC  GCC  CGC  TCG  CCC  AGC  CCC  AGC  ACA
CAG  CCC  TGG  GAA  CAT  GTG  AAT  GCC  ATC  CAG
GAA  GCC  CGG  CGT  CTC  CTG  AAC  CTG  AGT  AGA
GAC  ACT  GCT  GCT  GAG  ATG  AAT  GAA  ACA  GTA
GAA  GTC  ATC  TCA  GAA  ATG  TTT  GAC  CTC  CAG
GAG  CCG  ACC  TGC  CTA  CAG  ACC  CGC  CTG  GAG
CTG  TAC  AAG  CAG  GGC  CTG  CGG  GGC  AGC  CTC
ACC  AAG  CTC  AAG  GGC  CCC  TTG  ACC  ATG  ATG
GCC  AGC  CAC  TAC  AAA  CAG  CAC  TGC  CCT  CCA
ACC  CCG  GAA  ACT  TCC  TGT  GCA  ACC  CAG  ATT
ATC  ACC  TTT  GAA  AGT  TTC  AAA  GAG  AAC  CTG
AAG  GAC  TTT  CTG  CTT  GTC  ATC  CCC  TTT  GAC
TGC  TGG  GAG  CCA  GTC  CAG  GAG.
```

4. Expressionsvektor für die Expression von rekombinanter DNA nach einem der Ansprüche 1 bis 3, worin die für das Protein des Human-GM-CSF kodierende DNA an die Hefe-alpha-Faktor-Leitsequenz angeheftet ist.

5. Der Expressionsvektor pY α fGM-2 (ATCC 53157) für die Expression von rekombinanter DNA.

6. Eine Hefewirtszelle, welche mit einem Expressionsvektor für die Expression von rekombinanter DNA nach einem der Ansprüche 1 bis 5 transformiert worden ist.

**7.** Verfahren zur Herstellung eines Proteins des reifen, Human-Granulozyten-Makrophagen-Kolonien stimulierenden Faktors (GM-CSF), welches das Züchten von Hefewirtszellen nach Anspruch 6 in einem Kulturmedium und das Gewinnen des Human-GM-CSF-Proteins aus dem Kulturmedium umfaßt.

**Patentansprüche für folgenden Vertragsstaat: AT**

**1.** Verfahren zur Herstellung eines Expressionsvektors für die Expression von rekombinanter DNA, welcher einen Promotor enthält, der seinerseits in einer Hefewirtszelle die Expression einer Kodiersequenz von rekombinanter DNA steuert, welche Kodiersequenz:

(a) für ein Protein des reifen, Human-Granulozyten-Makrophagen-Kolonien stimulierenden Faktors (GM-CSF) kodiert, welches Protein N-terminale Alanin-Prolin-Reste aufweist, und welches Protein befähigt ist, das Wachstum von Human-Knochenmarks-Kolonien in dem Human-Knochenmarks-Kolonien-Assay zu stimulieren;

(b) sich an eine radioaktiv markierte, einzelsträngige DNA-Sonde hybridisiert, welche aus einem PstI-Hae-III-Fragment des Mäuse-GM-CSF-Gens besteht, welches Fragment den in der Fig. 1 dargestellten Nikleotiden 45 bis 400 entspricht, und zwar nach einer Über-Nacht-Hybridisierung in 6 x SSC bei 55° C und einem anschließenden Waschen mit 6 x SSC; und

(c) an ihrem 5'-Terminus an eine Leitsequenz fusioniert ist, welche aus einem entsprechenden Hefe-Pheromon-Gen stammt, und welche die Absonderung des reifen Human-GM-CSF-Proteins in das Kulturmedium nach Abspalten der Leitsequenz von den N-terminalen Alanin-Prolin-Resten steuert;. und welches Verfahren das Aneinanderkuppeln von aufeinanderfolgenden Nukleotiden umfaßt.

**2.** Verfahren nach Anspruch 1, wobei die Kodiersequenz von rekombinanter DNA für ein Protein des reifen Human-GM-CSF kodiert, welches Protein die Aminosäurensequenz:

| Ala | Pro | Ala | Arg | Ser | Pro | Ser | Pro | Ser | Thr |
|-----|-----|-----|-----|-----|-----|-----|-----|-----|-----|
| Gln | Pro | Trp | Glu | His | Val | Asn | Ala | Ile | Gln |
| Glu | Ala | Arg | Arg | Leu | Leu | Asn | Leu | Ser | Arg |
| Asp | Thr | Ala | Ala | Glu | Met | Asn | Glu | Thr | Val |
| Glu | Val | Ile | Ser | Glu | Met | Phe | Asp | Leu | Gln |
| Glu | Pro | Thr | Cys | Leu | Gln | Thr | Arg | Leu | Glu |
| Leu | Tyr | Lys | Gln | Gly | Leu | Arg | Gly | Ser | Leu |
| Thr | Lys | Leu | Lys | Gly | Pro | Leu | Thr | Met | Met |
| Ala | Ser | His | Tyr | Lys | Gln | His | Cys | Pro | Pro |
| Thr | Pro | Glu | Thr | Ser | Cys | Ala | Thr | Gln | Ile |
| Ile | Thr | Phe | Glu | Ser | Phe | Lys | Glu | Asn | Leu |
| Lys | Asp | Phe | Leu | Leu | Val | Ile | Pro | Phe | Asp |
| Cys | Trp | Glu | Pro | Val | Gln | Glu. | | | |

aufweist.

**3.** Verfahren nach Anspruch 2, wobei die Kodiersequenz von rekombinanter DNA aus der folgenden Nukleotidsequenz besteht:

```
GCA   CCC   GCC   CGC   TCG   CCC   AGC   CCC   AGC   ACA
CAG   CCC   TGG   GAA   CAT   GTG   AAT   GCC   ATC   CAG
GAA   GCC   CGG   CGT   CTC   CTG   AAC   CTG   AGT   AGA
GAC   ACT   GCT   GCT   GAG   ATG   AAT   GAA   ACA   GTA
GAA   GTC   ATC   TCA   GAA   ATG   TTT   GAC   CTC   CAG
GAG   CCG   ACC   TGC   CTA   CAG   ACC   CGC   CTG   GAG
CTG   TAC   AAG   CAG   GGC   CTG   CGG   GGC   AGC   CTC
ACC   AAG   CTC   AAG   GGC   CCC   TTG   ACC   ATG   ATG
GCC   AGC   CAC   TAC   AAA   CAG   CAC   TGC   CCT   CCA
ACC   CCG   GAA   ACT   TCC   TGT   GCA   ACC   CAG   ATT
ATC   ACC   TTT   GAA   AGT   TTC   AAA   GAG   AAC   CTG
AAG   GAC   TTT   CTG   CTT   GTC   ATC   CCC   TTT   GAC
TGC   TGG   GAG   CCA   GTC   CAG   GAG.
```

4. Verfahren nach einem der Ansprüche 1 bis 3, wobei die für das Protein des Human-GM-CSF kodierende DNA an die Hefe-alpha-Faktor-Leitsequenz angeheftet ist.

5. Verfahren zur Herstellung des Expressionsvektors pY α fGM-2 (ATCC 53157) für die Expression von rekombinanter DNA, welches Verfahren das Aneinanderkuppeln von aufeinanderfolgenden Nukleotiden umfaßt.

6. Verfahren zur Herstellung einer transformierten Hefewirtszelle, welches Verfahren das Transformieren einer Hefewirtszelle mit einem Expressionsvektor für die Expression von rekombinanter DNA nach einem der Ansprüche 1 bis 5 umfaßt.

7. Verfahren zur Herstellung eines Proteins des reifen, Human-Granulozyten-Makrophagen-Kolonien stimulierenden Faktors (GM-CSF), welches das Züchten von Hefewirtszellen, welche nach eins Verfahren gemäß dem Anspruch 6 hergestellt worden sind, in einem Kulturmedium und das Gewinnen des Human-GM-CSF-Proteins aus dem Kulturmedium umfaßt.

```
              10                      30  C  C   C     *      50                  A
        _____
        TGTCGGAGAGTCGTGGGTGGGCGAGTGGGATAATTGTTACCCGGCCTTGGAAGCATGTAGAGGCC
         SerGluSerArgGlyTrpAlaSerGlyIleThrValThrArgProTrpLysHisValGluAla
                                               ⟸_____

              70            G          90                A 110                130
        ATCAAAGAAGCCCTAAACCTCCTGGATGACATGCCTGTCACGTTGAATGAAGAGGTAGAAGTCGT
         IleLysGluAlaLeuAsnLeuLeuAspAspMetProValThrLeuAsnGluGluValGluValVa
        _____

                      150                  170                    190
        CTCTAACGAGTTCTCCTTCAAGAAGCTAACATGTGTGCAGACCCGCCTGAAGATATTCGAGCAGG
        lSerAsnGluPheSerPheLysLysLeuThrCysValGlnThrArgLeuLysIlePheGluGlnG
        _____

                      210                  230        ·           250
             __
        GTCTACCACGGGGCAATTTCACCAAACTCAAGGGCGCCTTGAACATGACAGCCAGCTACTACCAG
        lyLeuProArgGlyAsnPheThrLysLeuLysGlyAlaLeuAsnMetThrAlaSerTyrTyrGln
        _____GM-CSF cDNA Screening Probe Sequence_____

              270                  290                  310
        ACATACTGCCCCCCAACTCCGGAAACGGACTGTGAAACACAAGTCACCACCTATGCGGATTTCAT
        ThrTyrCysProProThrProGluThrAspCysGluThrGlnValThrThrTyrAlaAspPheIl

              330                  350                  370                  390
        AGACAGCCTTAAAACCTTTCTGACTGATATCCCCTTTGAATGCAAAAAACCAAGCCAAAAATGAG
        eAspSerLeuLysThrPheLeuThrAspIleProPheGluCysLysLysProSerGlnLysEnd
        _____

                      410                  430                  450
        GAAGCCCAGGCCAGCTCTGAATCCAGCTTCTCAGACTGCTGCTTTTGTGCCTGCGTAATGAGCCA
        _____⟹

        G      C     470                  490                  510  G
        AGAACTTGGAATTTCTGCCTTAAAGGGACCAAGAGATGTGGCACAGCCACAGTTGGAAGGCAGTA

              530            G         550                  570
        TAGCCCTCTGAAAACGCTAACTCAGCTTGGACAGCGGAAGACAAACGAGAGATATTTCTACTGA

              590                  610                  630                  650
        TAGGGACCATTATATTTATTTATATATTTATATTTTTTAAATATTATTTATTTATTTATTTATTT

                      670                  690                  710
        TTGGAACTCTATTTATTGAGAATGTCTTACCAGAATAATAAATTATTAAAACTTTAAAAAAAAAA
```

AAAAAAAAAA

**FIG. I** Nucleotide sequence (upper line) and amino acid sequence (lower line) of murine granulocyte-macrophage colony stimulating factor gene. Gough et al., 309 Nature (London) 763 (1984). Plasmid DNA fragment isolated with synthetic oligonucleotide probe extends from nucleotide Nos. 1 through 600. Overlined portions (nucleotide Nos. 1-28 and 201-203) contained in isolated plasmid DNA but not in Gough et al. Nucleotide No. 505 not contained in isolated plasmid DNA. Other differences in the nucleotide sequence of the isolated plasmid DNA are shown above corresponding nucleotides of the gene in Gough et al. The portion of the DNA fragment used to probe human cDNA library is indicated by

☀

↓ ┌─Sfa NI

```
           10      ↓ ┌        30            50            70
CTGCAGCATCTCTGCACCCGCCCGCTCGCCCAGCCCCAGCACACAGCCCTGGGAGCATGTGAATGCCATCCAGGAG

  CysSerIleSerAlaProAlaArgSerProSerProSerThrGlnProTrpGluHisValAsnAlaIleGlnGlu
                                10                            20
```

```
           90            110           130           150
GCCCGGCGTCTCCTGAACCTGAGTAGAGACACTGCTGCTGAGATGAATGAAACAGTAGAAGTCATCTCAGAAATG

  AlaArgArgLeuLeuAsnLeuSerArgAspThrAlaAlaGluMetAsnGluThrValGluValIleSerGluMet
                      30                            40
```

```
           170           190           210
TTTGACCTCCAGGAGCCGACCTGCCTACAGACCCGCCTGGAGCTGTACAAGCAGGGCCTGCGGGGCAGCCTCACC

  PheAspLeuGlnGluProThrCysLeuGlnThrArgLeuGluLeuTyrLysGlnGlyLeuArgGlySerLeuThr
      50                            60                            70
```

```
  230           250           270           290
AAGCTCAAGGGCCCCCTTGACCATGATGGCCAGCCACTACAAACAGCACTGCCCTCCAACCCCGGAAACTTCCTGT

  LysLeuLysGlyProLeuThrMetMetAlaSerHisTyrLysGlnHisCysProProThrProGluThrSerCys
                      80                            90
```

```
  310           330           350           370
GCAACCCAGATTATCACCTTTGAAAGTTTCAAAGAGAACCTGAAGGACTTTCTGCTTGTCATCCCCTTTGACTGCTGG

  AlaThrGlnIleIleThrPheGluSerPheLysGluAsnLeuLysAspPheLeuLeuValIleProPheAspCysTrp
      100                           110                           120
```

```
  390           410           430           450
GAGCCAGTCCAGGAGTGAGACCGGCCAGATGAGGCTGGCCAAGCCGGGGAGCTGCTCTCTCATGAAACAAGAGCTAG

  GluProValGlnGluEnd
```

```
           470           490           510           530
AAACTCAGGATGGTCATCTTGGAGGGACCAAGGGGTGGGCCACAGCCATGGTGGGAGTGGCCTGGACTGCCTGGCCA

                                        |
                                      Nco I
           550           570           590
CACTGACCTGATACAGGCATGGCAGAAGAATGGGATATTTATACTGACAAATACTGATATTATATATTATATTT
```

```
610           630           650
TAAATAATTTAATTTAATTTAATTTAATTGACTAATTACTATTATTACG
```

# FIG.2

Nucleotide sequence (upper line) and amino acid sequence (lower line) for human plasmid DNA pHG23 coding for granulocyte-macrophage colony stimulating factor gene. Mature protein begins at asterisk (*).

FIG.3

LANES

1  2  3  4  5  6

28S—

18S—

900
bases

**FIG.4** Hybridization of GM-CSF probe to Northern blots of RNA from human cells. Lane 1, 5 µg of total RNA from HUT-102 cells. Lane 2, 5 µg of total RNA from unstimulated peripheral blood T cells. Lane 3, 5 µg of total RNA from peripheral blood T cells stimulated with concanavalin A and phorbol myristic acetate. Lane 4, 1.5 µg of polyadenylated RNA from peripheral blood macrophages stimulated with lipopolysaccharide. Lane 5, 1.5 µg of polyadenylated RNA from the pancreatic carcinoma cell line 1420. Lane 6, 1.5 µg of polyadenylated RNA from the bladder carcinoma cell line 5637. The positions of 18S and 28S rRNA bands are indicated.

**FIG. 5** Hybridization of GM-CSF cDNA to Southern blots of human genomic DNA. Genomic DNA digested with: Hind III (lane 1), Eco RI (lane 2), Pst I (lane 3), Bgl II (lane 4).